# EUROPEAN PATENT APPLICATION

(11) **EP 2 295 401 A2**
(43) Date of publication of application: **16.03.2011**
(21) Application number: 10177438.8
(22) Date of filing: 14.03.2007
(51) Int. Cl.: C07C 231/20, C07C 237/04, C07D 209/52, C07D 403/12, C07D 403/14, C07D 498/10, A61K 31/498, A61K 31/422, A61K 31/403, A61P 31/12

(54) **Deuterated hepatitis C protease inhibitors**

(30) Priority: 16.03.2006 US 782788 P; 16.03.2006 US 782976 P; 15.09.2006 US 844771 P
(62) Divisional of application: 07753142.4
(71) Applicant: VERTEX PHARMACEUTICALS INCORPORATED, Cambridge, MA 02139-4242 (US)
(72) Inventor: Perni, Robert B., Cambridge MA 02139-4242 (US); Chen, Minzhang, Acton MA 01720 (US); Jung, Young Chun, Lexington MA 02420 (US); Forslund, Raymond E., Natick MA 01760 (US); Tanoury, Gerald, J., Hudson MA 01749 (US); Bennani, Youssef, Boston MA 02116 (US); Zlokarnik, Gregor, La Jolla CA 92037 (US); Maltais, Francois, Tewksbury MA 01876 (US)
(74) Representative: Cohausz & Florack

(57) **Abstract**

A deuterated α-ketoamido steric specific compound of the formular (I), wherin D denotes a deuterium atom on a steric specific carbon atom.

## Description

### CROSS-REFERENCE

This application claims the benefits of U.S. Provisional Application Serial No. 60/782,778, filed March 16, 2006, U.S. Provisional Application Serial No. 60/782,976, filed March 16, 2006, and U.S. Provisional Application Serial No. 60/844,771, filed September 15, 2006.

### BACKGROUND OF THE INVENTION

Infection by hepatitis C virus ("HCV") is a compelling human medical problem. HCV is recognized as the causative agent for most cases of non-A, non-B hepatitis, with an estimated human sero-prevalence of 3% globally [A. Alberti et al., "Natural History of Hepatitis C," J. Hepatology, 31., (Suppl. 1), pp. 17-24 (1999)]. Nearly four million individuals may be infected in the United States alone [M.J. Alter et al., "The Epidemiology of Viral Hepatitis in the United States, Gastroenterol. Clin. North Am., 23, pp. 437-455 (1994); M. J. Alter "Hepatitis C Virus Infection in the United States," J. Hepatology, 31., (Suppl. 1), pp. 88-91 (1999)].

Upon first exposure to HCV only about 20% of infected individuals develop acute clinical hepatitis while others appear to resolve the infection spontaneously. In almost 70% of instances, however, the virus establishes a chronic infection that persists for decades [S. Iwarson, "The Natural Course of Chronic Hepatitis," FEMS Microbiology Reviews, 14, pp. 201-204 (1994); D. Lavanchy, "Global Surveillance and Control of Hepatitis C," J. Viral Hepatitis, 6, pp. 35-47 (1999)]. This usually results in recurrent and progressively worsening liver inflammation, which often leads to more severe disease states such as cirrhosis and hepatocellular carcinoma [M.C. Kew, "Hepatitis C and Hepatocellular Carcinoma", FEMS Microbiology Reviews, 14, pp. 211-220 (1994); I. Saito et. al., "Hepatitis C Virus Infection is Associated with the Development of Hepatocellular Carcinoma," Proc. Natl. Acad. Sci. USA, 87, pp. 6547-6549 (1990)]. Unfortunately, there are no broadly effective treatments for the debilitating progression of chronic HCV.

The HCV genome encodes a polyprotein of 3010-3033 amino acids [Q.L. Choo, et. al., "Genetic Organization and Diversity of the Hepatitis C Virus." Proc. Natl. Acad. Sci. USA, 88, pp. 2451-2455 (1991); N. Kato et al., "Molecular Cloning of the Human Hepatitis C Virus Genome From Japanese Patients with Non-A, Non-B Hepatitis," Proc. Natl. Acad. Sci. USA, 87, pp. 9524-9528 (1990); A. Takamizawa et. al., "Structure and Organization of the Hepatitis C Virus Genome Isolated From Human Carriers," J. Virol., 65, pp. 1105-1113 (1991)]. The HCV nonstructural (NS) proteins are presumed to provide the essential catalytic machinery for viral replication. The NS proteins are derived by proteolytic cleavage of the polyprotein [R. Bartenschlager et. al., "Nonstructural Protein 3 of the Hepatitis C Virus Encodes a Serine-Type Proteinase Required for Cleavage at the NS3/4 and NS4/5 Junctions," J. Virol., 67, pp. 3835-3844 (1993); A. Grakoui et. al., "Characterization of the Hepatitis C Virus-Encoded Serine Proteinase: Determination of Proteinase-Dependent Polyprotein Cleavage Sites," J. Virol., 67, pp. 2832-2843 (1993); A. Grakoui et. al., "Expression and Identification of Hepatitis C Virus Polyprotein Cleavage Products," J. Virol., 67, pp. 1385-1395 (1993); L. Tomei et. al., "NS3 is a serine protease required for processing of hepatitis C virus polyprotein", J. Virol., 67, pp. 4017-4026 (1993)].

The HCV NS protein 3 (NS3) contains a serine protease activity that helps process the majority of the viral enzymes, and is thus considered essential for viral replication and infectivity. It is known that mutations in the yellow fever virus NS3 protease decrease viral infectivity [Chambers, T.J. et. al., "Evidence that the N-terminal Domain of Nonstructural Protein NS3 From Yellow Fever Virus is a Serine Protease Responsible for Site-Specific Cleavages in the Viral Polyprotein", Proc. Natl. Acad. Sci. USA, 87, pp. 8898-8902 (1990)]. The first 181 amino acids of NS3 (residues 1027-1207 of the viral polyprotein) have been shown to contain the serine protease domain of NS3 that processes all four downstream sites of the HCV polyprotein [C. Lin et al., "Hepatitis C Virus NS3 Serine Proteinase: Trans-Cleavage Requirements and Processing Kinetics", J. Virol., 68, pp. 8147-8157 (1994)].

The HCV NS3 serine protease and its associated cofactor, NS4A, help process all of the viral enzymes, and is thus considered essential for viral replication. This processing appears to be analogous to that carried out by the human immunodeficiency virus aspartyl protease, which is also involved in viral enzyme processing. HIV protease inhibitors, which inhibit viral protein processing, are potent antiviral agents in man, indicating that interrupting this stage of the viral life cycle results in therapeutically active agents. Consequently HCV NS3 serine protease is also an attractive target for drug discovery.

There are not currently any satisfactory anti-HCV agents or treatments. Until recently, the only established therapy for HCV disease was interferon treatment. However, interferons have significant side effects [M. A. Wlaker et al., "Hepatitis C Virus: An Overview of Current Approaches and Progress," DDT, 4, pp. 518-29 (1999); D. Moradpour et al., "Current and Evolving Therapies for Hepatitis C," Eur. J. Gastroenterol. Hepatol.,11, pp. 1199-1202 (1999); H. L. A. Janssen et al. "Suicide Associated with Alfa-Interferon Therapy for Chronic Viral Hepatitis," J. Hepatol., 21, pp. 241-243 (1994); P.F. Renault et al., "Side Effects of Alpha Interferon," Seminars in Liver Disease, 9, pp. 273-277. (1989)] and induce long term remission in only a fraction (∼ 25%) of cases [O. Weiland, "Interferon Therapy in Chronic Hepatitis C Virus Infection", FEMS Microbiol. Rev., 14, pp. 279-288 (1994)]. Recent introductions of the pegylated forms of interferon (PEG-INTRON® and PEGASYS®) and the combination therapy of ribavirin and pegylated interferon (REBETROL®) have resulted in only modest improvements in remission rates and only partial reductions in side effects. Moreover, the prospects for effective anti-HCV vaccines remain uncertain.

Thus, there is a need for more effective anti-HCV therapies. Such inhibitors would have therapeutic potential as protease inhibitors, particularly as serine protease inhibitors, and more particularly as HCV NS3 protease inhibitors. Specifically, such compounds may be useful as antiviral agents, particularly as anti-HCV agents.

It was recently discovered that deuterium incorporation in a compound will reduce the rate of epimerization via a deuterium isotope effect, thus enhancing the concentration of the active isomers in vivo relative to its non-deuterated analogs.

### SUMMARY OF THE INVENTION

The present invention relates to deuterated compounds of formula (I) 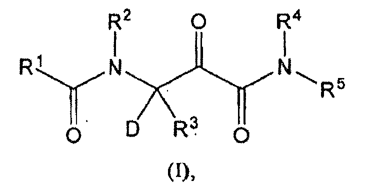 as well as pharmaceutically acceptable salts, prodrugs, and solvates thereof. In formula (I), D denotes a deuterium atom.

Referring to formula (I),
D denotes a deuterium atom;
R' is 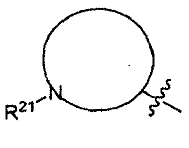 in which 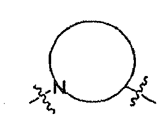 is an optionally substituted monocyclic azaheterocyclyl or optionally substituted multicyclic azaheterocyclyl, or optionally substituted.multicyclic azaheterocyclenyl wherein the unsaturatation is in the ring distal to the ring bearing the R²¹ moiety and to which the -C(O)-N(R²)-CDR³-C(O)-C(O)-NR⁴R⁵ moiety is attached;
R²¹ is Q³-W³-Q²-W²-Q¹; wherein
Each of W² and W³ is independently a bond, -CO-, -CS-, -C(O)N(Q⁴)-, -CO₂-, -O-, -N(Q⁴)-C(O)-N(Q⁴)-, -N(Q⁴)-C(S)-N(Q⁴)-, -OC(O)NQ⁴-, -S-, -SO-, -SO₂-,- - N(Q⁴)-, -N(Q⁴)SO₂-, -N(Q⁴)SO₂N(Q⁴)-, and hydrogen when any of W² and W³ is the terminal group;
Each of Q¹, Q², and Q³ is independently a bond, an optionally substituted aliphatic, an optionally substituted heteroaliphatic, an optionally substituted cycloaliphatic, an optionally substituted aryl, an optionally substituted heteroaryl, an optionally substituted aralkyl, or an optionally substituted heteraaralkyl; or hydrogen when any of Q³, Q², or Q¹ is the terminal group, provided that Q² is not a bond when both W³ and W² are present; and
Each of R², R³, and R⁴, independently, is H or a C₁₋₆ alkyl;
R⁵ is H, alkyl, cycloalkyl, aryl optionally substituted with 1-4 alkyl groups, alkylaryl, aryl, amino optionally substituted with 1 or 2 alkyl groups; and
R²¹ is Q³-W³-Q²-W²-Q¹; wherein each of W² and W³ is independently a bond, -CO-, -CS-, -C(O)N(Q⁴)-, -CO₂-, -O-, -N(Q⁴)-C(O)-N(Q⁴)-, -N(Q⁴)-C(S)-N(Q⁴)-, -OC(O)NQ⁴-, -S-, -SO-, -SO₂-, -N(Q⁴)-, -N(Q⁴)SO₂-, -N(Q⁴)SO₂N(Q⁴)-, and hydrogen when any of W² and W³ is the terminal group; each of Q¹, Q², and Q³ is independently a bond, an optionally substituted aliphatic, an optionally substituted heteroaliphatic, an optionally substituted cycloaliphatic, an optionally substituted aryl, an optionally substituted heteroaryl, an optionally substituted aralkyl, or an optionally substituted heteroaralkyl; or hydrogen when any of Q³, Q², or Q¹ is the terminal group, provided that Q² is not a bond when both W³ and W² are present.

In some embodiments, R¹ is 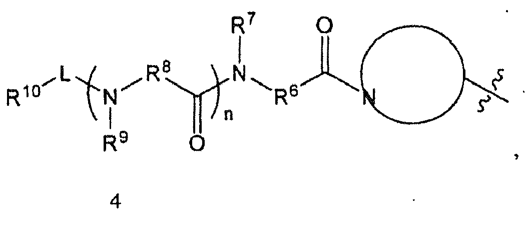 in
which
each of R⁶ and R⁸ is independently
a bond; or
optionally substituted (1,1- or 1,2-)cycloalkylene; or
optionally substituted (1,1-or 1,2-)heterocyclylene; or
methylene or ethylene, substituted with one substituent selected from the group consisting of an optionally substituted aliphatic group, an optionally substituted cyclic group and an optionally substituted aromatic group, and wherein the methylene or ethylene is further optionally substituted with an aliphatic group substituent;
each of R⁷, R⁹, and R¹¹ is independently hydrogen or optionally substituted aliphatic group;
R¹⁰ is an optionally substituted aliphatic group, optionally substituted cyclic group or optionally substituted aromatic group;
L is -C(O)-, -OC(O)-, -NR¹¹C(O)-, -S(O)₂-, -NR¹¹ S(O)₂-, or a bond; and
n is 0 or 1.

In some embodiments, n is 1.

In some embodiments, R⁶ is methylene substituted with one substituent selected from the group consisting of an optionally substituted aliphatic group, an optionally substituted cyclic group, and an optionally substituted aromatic group.

In some embodiments, R⁶ is methylene substituted with isobutyl.

In some embodiments, R⁷ is hydrogen.

In some embodiments, R⁸ is methylene substituted with one substituent selected from the group consisting of an optionally substituted aliphatic group, an optionally substituted cyclic group, and an optionally substituted aromatic group. In some other embodiments, R⁸ is methylene substituted with an optionally substituted cyclic group. In still some other embodiments, R⁸ is methylene substituted with cyclohexyl.

In some embodiments, R⁹ is hydrogen.

In some embodiments, L is -CO-.

In some embodiments, R¹⁰ is an optionally substituted aromatic group.

In some embodiments, R¹⁰ is selected from the group
consisting of

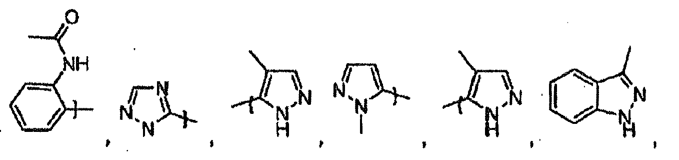

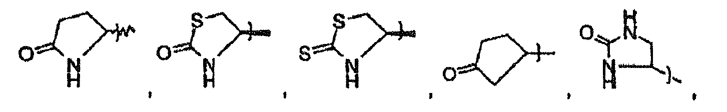

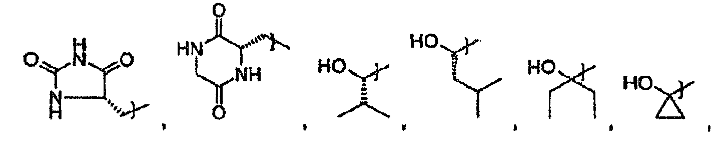

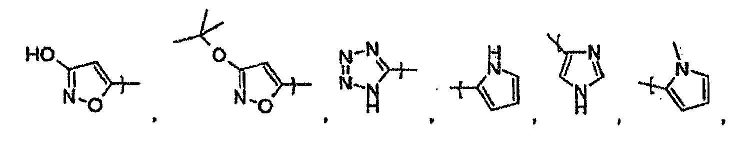

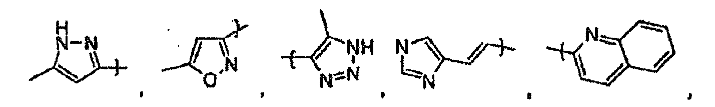

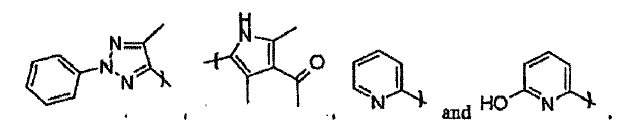

In some embodiments, R¹⁰ is optionally substituted pyrazinyl (e.g., 2-pyrazinyl).

In some embodiments, 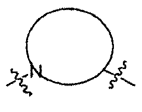 is a substituted monocyclic azaheterocyclyl.

In some other embodiments, 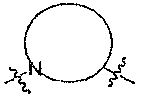 is pyrrolidinyl substituted at the 3-position carbon atom with heteroaryloxy, wherein the heteroaryl is further optionally substituted with 1-4 halo groups.

In some embodiments,

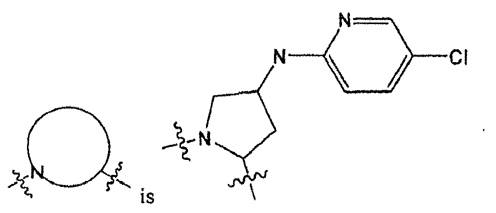

In some embodiments, 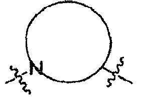 is an optionally substituted multicyclic azaheterocyclyl.

In another embodiment, 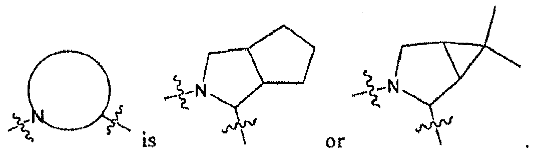 In some embodiments,

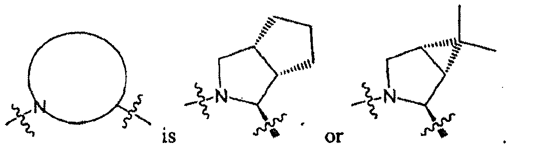

In another embodiment, R² is hydrogen, each of R⁴ and R⁵ independently is hydrogen or cyclopropyl. In another embodiment, R³ is propyl. In another embodiment, n is 0. In another embodiment, L is -NR¹¹ C(O)- and R¹¹ is hydrogen. In another embodiment, R¹⁰ is an optionally substituted aliphatic group. In another embodiment, R¹⁰ is t-butyl. In another embodiment, the compound is 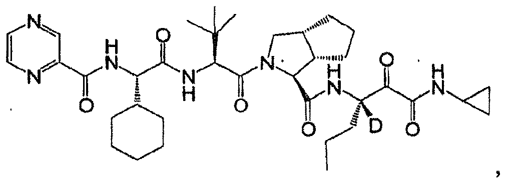 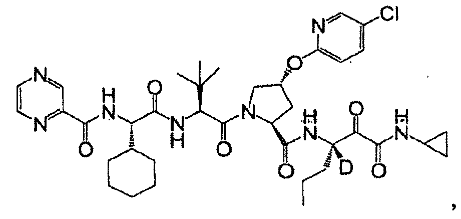 or

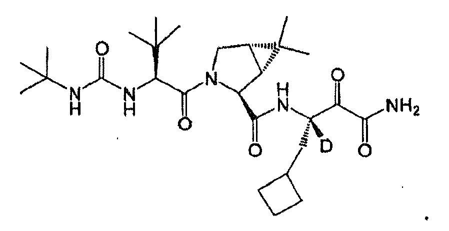

In some embodiments, R¹ is 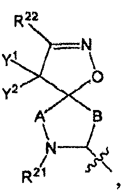 in which
A is -(CHX¹)a-;
B is -(CHX²)b-;
a is 0 to 3;
b is 0 to 3, provided that a + b is 2 or 3;
each of X¹ and X² is independently selected from hydrogen, optionally substituted C₁. ₄ aliphatic, and optionally substituted aryl;
each of Y¹ and Y² is independently hydrogen, optionally substituted aliphatic, optionally substituted aryl, amino, or -OQ⁴; wherein each Q⁴ is independently hydrogen or an optionally substituted aliphatic;
R²² is an optionally substituted aliphatic, an optionally substituted heteroaliphatic, an optionally substituted cycloaliphatic, an optionally substituted heterocycloaliphatic, an optionally substituted aryl, or an optionally substituted heteroaryl. In some embodiments, R²¹ is optionally substituted alkylcarbonyl.

The moiety 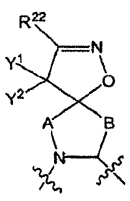 includes all of its stereospecific enantiomers, e.g., 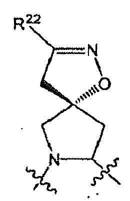 (when A and B are both CH₂, and Y¹ and Y² are both H).

In some embodiments, R²¹ is aminoalkylcarbonyl, haloalkylcarbonyl, arylalkylcarbonyl, arylalkylcarbonyl, cycloaliphaticalkylcarbonyl, or heterocycloaliphaticalkylcarbonyl, each of which is optionally substituted with 1-3 substituents. In some embodiments, R²¹ is heterocycloalkyl-oxycarbonylamino-alkylcarbonyl, heteroaryl-carbonylamino-alkyl-carbonylamino-alkyl-carbonyl, bicycloaryl-sulfonylamino-alkylcarbonyl, aryl-alkoxy-carbonylamino-alkyl-carbonyl, alkyl-carbonylamino-alkyl-carbonyl, aliphatic-oxycarbonylamino-alkyl-carbonyl, cycloaliphatic-alkyl-aminocarbonylamino-alkyl-carbonyl, heteroaryl-carbonylamino-alkyl-carbonylamino-alkyl-carbonyl, alkyl-aminocarbonylamino-alkyl-carbonyl, or bicycloaryl-aminocarbonylamino-alkyl-carbonyl, each of which is optionally substituted with 1-3 substituents. In some embodiments, R²² is an optionally substituted aliphatic, optionally substituted heteroaliphatic, optionally substituted cycloaliphatic, optionally substituted heterocycloaliphatic, optionally substituted aryl, or optionally substituted heteroaryl. In some embodiments, R²² is optionally substituted phenyl, optionally substituted naphthyl, optionally substituted anthracenyl, optionally substituted naphthalene, or optionally substituted anthracene. In some embodiments, each of X¹, X², Y¹ and Y² is hydrogen, each of a and b is 1.

In some embodiments, R²¹ is an optionally substituted alkylcarbonyl.

In some embodiments, R²¹ is an aminoalkylcarbonyl, haloalkylcarbonyl, arylalkylcarbonyl, arylalkylcarbonyl, cycloaliphaticalkylcarbonyl, or heterocycloaliphaticalkylcarbonyl, each of which is optionally substituted with 1-3 substituents.

In some embodiments, R²¹ is heterocycloalkyl-oxycarbonylamino-alkylcarbonyl, heteroaryl-carbonylamino-alkyl-carbonylamino-alkyl-carbonyl, bicycloaryl-sulfonylamino-alkylcarbonyl, aryl-alkoxy-carbonylamino-alkyl-carbonyl, alkyl-carbonylamino-alkyl-carbonyl, aliphatic-oxycarbonylamino-alkyl-carbonyl, cycloaliphatic-alkyl-aminocarbonylamino-alkyl-carbonyl, cycloaliphatic-alkyl-carbonylamino-alkyl-carbonyl, heteroaryl-carbonylamino-alkyl-carbonylamino-alkyl-carbonyl, alkyl-aminocarbonylamino-alkyl-carbonyl, or bicycloaryl-aminocarbonylamino-alkyl-carbonyl, each of which is optionally substituted with 1-3 substituents.

In some embodiments, R²² is an optionally substituted aliphatic, optionally substituted heteroaliphatic, optionally substituted cycloaliphatic, optionally substituted heterocycloaliphatic, optionally substituted aryl, or optionally substituted heteroaryl.

In some embodiments, R²² is optionally substituted phenyl, optionally substituted naphthyl, optionally substituted anthracenyl, optionally substituted naphthalene, or optionally substituted anthracene.

In some embodiments, each of X¹, X², Y¹, and Y² is hydrogen, each of a and b is 1.

In some embodiments, R²² is an optionally substituted aliphatic, optionally substituted heteroaliphatic, optionally substituted cycloaliphatic, optionally substituted heterocycloaliphatic, optionally substituted aryl, or optionally substituted heteroaryl.

In an embodiment, the compound is

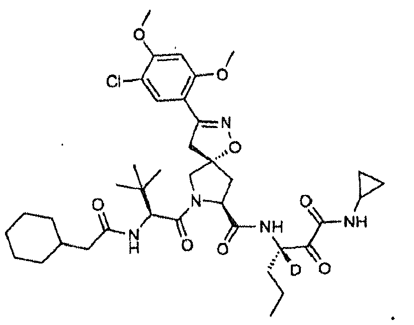

The deuterated compounds of this invention undergo slower epimerization than its non-deuterated counterparts. As shown below, the deuterated compound 1 very slowly converts to a non-deuterated intermediate which then converts to epimers 2 and 3. The epimers 2 and 3 then maintain in an equilibrium, which further slows the epimerization of the deuterated compound 1. 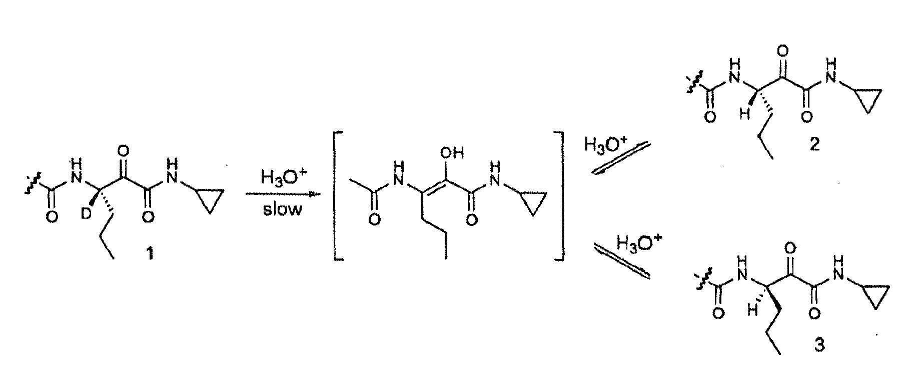 As a result of their slow epimerization, the deuterated compounds of this invention can enhance the concentration of the active isomers *in vivo* relative to its non-deuterated analogs.

In some embodiments, the deuterium enrichment is at least 50% in the compounds of this invention. In some embodiments, the deuterium enrichment is at least 80% in the compounds of this invention. In some embodiments, the deuterium enrichment is at least 90% in the compounds of this invention. In some embodiments, the deuterium enrichment is at least 99% in the compounds of this invention.

The invention also relates to a pharmaceutical composition comprising a pharmaceutically acceptable carrier and a compound of formula (I) or any of its embodiments described above.

The invention also relates to a method for increasing the concentration of the active isomer of a pharmaceutical agent in *vivo,* comprising administering to a patient in need thereof a deuterated isomer of the pharmaceutical agent in an amount sufficient to confer the pharmaceutical effect.

The invention also relates to a method for enhancing the bioavailability of a compound, comprising replacing a hydrogen atom that is bonded to a steric carbon atom in the compound with a deuterium atom. In one embodiment, the deuterated compound is of formula (I) or any of its embodiments described above.

The invention also relates to a method for inhibiting HCV protease, comprising contacting HCV protease with a deuterated compound of formula (I) or any of its embodiments described above.

The invention also relates to a method for treating a patient suffering from HCV infection or a condition mediated by HCV protease, comprising administering to the patient a pharmaceutically effective amount of a deuterated compound of formula (I) or any of its embodiments described above.

Also within the scope of this invention is a process for preparing an optically enriched compound of Formula 1, in which 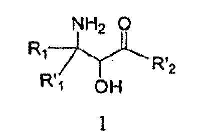 the carbon atoms alpha and beta to the carboxy group are stereocenters;
R₁ is independently H, optionally substituted aliphatic, optionally substituted cycloaliphatic, optionally substituted arylaliphatic, optionally substituted heteroaliphatic or optionally substituted heteroarylaliphatic;
R'₁ is deuterium,
R'₂ is -NHR₂ or -OE;
R₂ is H, an optionally substituted aliphatic, optionally substituted cycloaliphatic, optionally substituted arylaliphatic, optionally substituted heteroaliphatic or optionally substituted heteroarylaliphatic; and
E is a C₁₋₆ alkyl or benzyl;

The method includes the steps of:
a) forming a salt of a compound of Formula 1, and
b) crystallizing said salt to give a compound of greater than 55% enantiomeric excess.

In some embodiments, R₁ is C₁₋₆ alkyl, and R'₂ is -NHR₂ wherein R₂ is a C₁₋₆ alkyl or C₁₋₆ cycloalkyl. In some embodiments, R₁ is propyl and R₂ is cyclopropyl.

In some embodiments, the method further includes aminating a compound of Formula ii 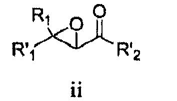 with an aminating reagent to provide a compound of Formula iii 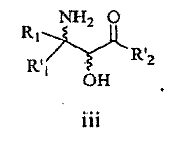 In still some embodiments, the aminating reagent is an azide salt and the intermediate azido compound is reduced by hydrogenation.

In some embodiments, the method further includes oxidizing an unsaturated compound of Formula i 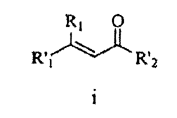 wherein R'₂ is -NHR₂ or -OE, wherein E is C₁₋₅ alkyl or optionally substituted benzyl, with an oxidizing reagent to provide a compound of Formula ii.

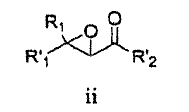

In some further embodiments, the oxidizing reagent comprises t-butyl hydroperoxide. In some further embodiments, the oxidizing reagent further includes a chiral reagent. In some further embodiments, the oxidizing reagent is a mixture of samarium (III) isopropoxide, triphenyl arsine oxide, S-(-)1,1'-bi-2-naphthol and 4 Å molecular sieves. In some further embodiments, the oxidizing reagent comprises urea-hydrogen peroxide in the presence of trifluoroacetic anhydride.

In some further embodiments, the method further includes hydrolyzing the compound of Formula ii to give an acid and then converting the acid to an amide compound of Formula ii wherein R'₂ is -NHR₂.

Still within the scope of this invention is a process for preparing a compound of Formula 1 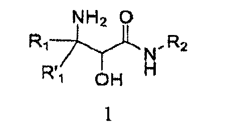 wherein:
R₁ is H, optionally substituted aliphatic, optionally substituted cycloaliphatic, optionally substituted arylaliphatic, optionally substituted heteroaliphatic or optionally substituted heteroarylaliphatic;
R'₁ is deuterium,
R₂ is H, optionally substituted aliphatic, optionally substituted cycloaliphatic, optionally substituted arylaliphatic, optionally substituted heteroaliphatic or optionally substituted heteroarylaliphatic; and
the compound of Formula 1 has an enantiomeric excess of greater than 55%.
The method includes the steps of:
a) oxidation of an unsaturated compound of Formula i 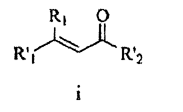 to provide a compound of formula ii 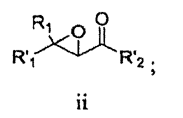
b) reacting a compound of Formula ii with an aminating reagent to provide a compound of Formula iii 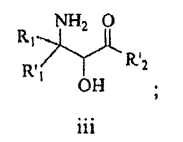
c) forming a salt of a compound of Formula iii with an optically active organic acid; and
d) crystallizing said salt to give a compound of greater than 55% enantiomeric excess.

In some embodiments, the compound of Formula 1 is (2S,3S)-3-amino-3-deutero-N-cyaopropyl-2-hydroxyhexanamide. In some embodiments, the organic acid is L-tartaric acid or deoxycholic acid.

Also within the scope of this invention is a process for preparing an optically enriched compound of Formula 1: 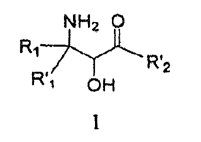 wherein:
the carbon atoms alpha and beta to the carboxy group are stereocenters;
R₁ is independently H, optionally substituted aliphatic, optionally substituted cycloaliphatic, optionally substituted arylaliphatic, optionally substituted heteroaliphatic or optionally substituted heteroarylaliphatic;
R'₁ is deuterium such that the deuterium enrichment is at least 50%;
R'₂ is -NHR₂ or -OE;
R₂ is H, optionally substituted aliphatic, optionally substituted cycloaliphatic, optionally substituted arylaliphatic, optionally substituted heteroaliphatic or optionally substituted heteroarylaliphatic; and
E is C₁₋₆ alkyl or benzyl.
The method includes the steps of : a) forming a salt of a compound of Formula 1, and b) crystallizing said salt to give a compound of greater than 55% enantiomeric excess.

In some embodiments, R₁ is C₁₋₆ alkyl, and R'₂ is -NHR₂ wherein R₂ is a C₁₋₆ alkyl or C₁₋₆ cycloalkyl. In some embodiments, R₁ is propyl and R₂ is cyclopropyl.

In some embodiments, the method further includes the step of aminating a compound of Formula ii 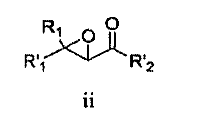 with an aminating reagent to provide a compound of Formula iii

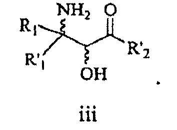

In some embodiments, the aminating reagent is an azide salt and the intermediate azido compound is reduced by hydrogenation.

In some embodiments, the method further includes the step of oxidizing an unsaturated compound of Formula i 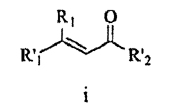 wherein R'₂ is -NHR₂ or -OE, wherein E is C₁₋₅ alkyl or optionally substituted benzyl, with an oxidizing reagent to provide a compound of Formula ii. 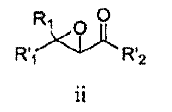 In some embodiments, the oxidizing reagent comprises t-butyl hydroperoxide. In some further embodiments, the oxidizing reagent further a chiral reagent. In some embodiments, the oxidizing reagent is a mixture of samarium (III) isopropoxide, triphenyl arsine oxide, S-(-) 1, 1'-bi-2-naphthol and 4 Å molecular sieves. In some embodiments, the oxidizing reagent comprises urea-hydrogen peroxide in the presence of trifluoroacetic anhydride.

In some embodiments, R'2 is -OE. In some embodiments, R'₂ is -NHR₂.

In some embodiments, the method further includes hydrolyzing the compound of Formula ii to give an acid and then converting the acid to an amide compound of Formula ii wherein R'₂ is -NHR₂.

In some embodiments, the method further includes oxidizing a compound of Formula iv 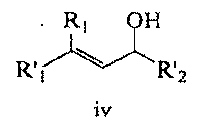 to give the compound of Formula ii. In some instance, the oxidation is conducted by using manganese dioxide.

In some embodiments, the method further includes reducing a compound of Formula v 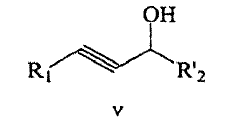 to give the compound of Formula iv. In some instance, the compound is reduced with Red-A1® and then quenched with deuterium oxide. As known in the art, "Red-A1®" refers to the compound [(CH₃OCH₂OCH₂)₂AlH₂]Na which is commercial available, generally as a solution in toluene (e.g., 70% W/W). For more information about Red-A1®, see, e.g., Bates R.W. et al., Tetrahedron, 1990, 46, 4063.

Still within the scope of this invention is a process for preparing a compound of Formula 1 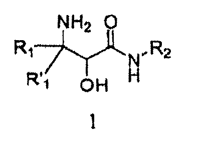 wherein:
R₁ is H, optionally substituted aliphatic, optionally substituted cycloaliphatic, optionally substituted arylaliphatic, optionally substituted heteroaliphatic or optionally substituted heteroarylaliphatic;
R'₁ is deuterium,
R₂ is H, optionally substituted aliphatic, optionally substituted cycloaliphatic, optionally substituted arylaliphatic, optionally substituted heteroaliphatic or optionally substituted heteroarylaliphatic; and
the compound of Formula 1 has an enantiomeric excess of greater than 55%. The method includes the steps of :
   a) oxidation of an unsaturated compound of Formula i 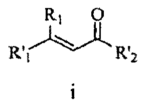 to provide a compound of formula ii 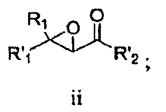
   b) reacting a compound of Formula ii with an aminating reagent to provide a compound of Formula iii 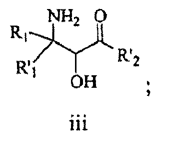
   c) forming a salt of a compound of Formula iii with an optically active organic acid; and
   d) crystallizing said salt to give a compound of greater than 55% enantiomeric excess.

In some embodiments, the compound of Formula 1 is (2S,3S)-3-amino-3-deutero-N-cyclopropyl-2-hydroxyhexanamide. In some embodiments, the organic acid is L-tartaric acid or deoxycholic acid.

### DETAILED DESCRIPTION OF THE INVENTION

### I. Definitions

### A. Terms

As used herein, the term "aliphatic" encompases alkyl, alkenyl and alkynyl.

As used herein, an "alkyl" group refers to a saturated aliphatic hydrocarbon group containing 1-8 (e.g., 1-6 or 1-4) carbon atoms. An alkyl group can be straight or branched. Examples of an alkyl group include, but are not limited to, methyl, ethyl, propyl, isopropyl, butyl, isobutyl, sec-butyl, tert-butyl, n-pentyl, n-heptyl, and 2-ethylhexyl. An alkyl group can be optionally substituted with one or more substituents such as alkoxy; cycloalkyloxy, heterocycloalkyloxy, aryloxy, heteroaryloxy, aralkyloxy, heteroarylalkoxy, amino, nitro, carboxy, cyano, halo, hydroxy, sulfo, mercapto, alkylsulfanyl, alkylsulfinyl, alkylsulfonyl, aminocarbonyl, alkylcarbonylamino, cycloalkylcarbonylamino, cycloalkyl-alkylcarbonylamino, arylcarbonylamino, aralkylcarbonylamino, heterocycloalkyl-carbonylamino, heterocycloalkyl-alkylcarbonylamino, heteroarylcarbonylamino, heteroaralkylcarbonylamino, urea, thiourea, sulfamoyl, sulfamide, alkoxycarbonyl, or alkylcarbonyloxy.

As used herein, an "alkenyl" group refers to an aliphatic carbon group that contains 2-8 (e.g., 2-6 or 2-4) carbon atoms and at least one double bond. Like an alkyl group; an alkenyl group can be straight or branched. Examples of an alkenyl group include, but are not limited to, allyl, isoprenyl, 2-butenyl, and 2-hexenyl. An alkenyl group can be optionally substituted with one or more substituents such as alkoxy, cycloalkyloxy, heterocycloalkyloxy, aryloxy, heteroaryloxy, aralkyloxy, heteroarylalkoxy, amino, nitro, carboxy, cyano, halo, hydroxy, sulfo, mercapto, alkylsulfanyl, alkylsulfinyl, alkylsulfonyl, aminocarbonyl, alkylcarbonylamino, cycloalkylcarbonylamino, cycloalkyl-alkylcarbonylamino, arylcarbonylamino, aralkylcarbonylamino, heterocycloalkyl-carbonylamino, heterocycloalkyl-alkylcarbonylamino, heteroarylcarbonylamino, heteroaralkylcarbonylamino, urea, thiourea, sulfamoyl, sulfamide, alkoxycarbonyl, or alkylcarbonyloxy.

As used herein, an "alkynyl" group refers to an aliphatic carbon group that contains 2-8 (e.g., 2-6 or 2-4) carbon atoms and has at least one triple bond. An alkynyl group can be straight or branched. Examples of an alkynyl group include, but are not limited to, propargyl and butynyl. An alkynyl group can be optionally substituted with one or more substituents such as alkoxy, cycloalkyloxy, heterocycloalkyloxy, aryloxy, heteroaryloxy, aralkyloxy, heteroarylalkoxy, amino, nitro, carboxy, cyano, halo, hydroxy, sulfo, mercapto, alkylsulfanyl, alkylsulfinyl, alkylsulfonyl, aminocarbonyl, alkylcarbonylamino, cycloalkylcarbonylamino, cycloalkyl-alkylcarbonylamino, arylcarbonylamino, aralkylcarbonylamino, heterocycloalkyl-carbonylamino, heterocycloalkyl-alkylcarbonylamino, heteroarylcarbonylamino, heteroaralkylcarbonylamino, urea, thiourea, sulfamoyl, sulfamide, alkoxycarbonyl, or alkylcarbonyloxy.

As used herein, an "amino" group refers to -NR^{X}R^{Y} wherein each of R^{x} and R^{Y} is independently hydrogen, alkyl, cycloalkyl, (cycloalkyl)alkyl, aryl, aralkyl, heterocycloalkyl, (heterocycloalkyl)alkyl, heteroaryl, or heteroaralkyl. When the term "amino" is not the terminal group (e.g., alkylcarbonylamino), it is represented by -NR^{x}-. R^{x} has the same meaning as defined above.

As used herein, an "aryl" group refers to phenyl, naphthyl, or a benzofused group having 2 to 3 rings. For example, a benzofused group includes phenyl fused with one or two C₄-₈ cycloaliphatic moieties, e.g., 1, 2, 3; 4-tetrahydronaphthyl, indanyl, dihydroindanyl, or fluorenyl. An aryl is optionally substituted with one or more substituents such as alkyl (including carboxyalkyl, hydroxyalkyl, and haloalkyl such as trifluoromethyl), alkenyl, alkynyl, cycloalkyl, (cycloalkyl)alkyl, heterocycloalkyl, (heterocycloalkyl)alkyl, aryl, heteroaryl, alkoxy, cycloalkyloxy, heterocycloalkyloxy, aryloxy, heteroaryloxy, aralkyloxy, heteroaralkyloxy, aroyl, heteroaroyl, amino, nitro, carboxy, alkoxycarbonyl, alkylcarbonyloxy, aminocarbonyl, alkylcarbonylamino, cycloalylcarbonylamino, (cycloalky)alkylcarbonylamino, arylcarbonylamino, aralkylcarbonylamino, (heterocycloalkyl)carbonylamino, (heterocycloalkyl)alkylcarbonylamino, heteroarylcarbonylamino, heteroaralkylcarbonylamino, cyano, halo, hydroxy, acyl, mercapto, alkylsulfanyl, sulfoxy, urea, thiourea, sulfamoyl, sulfamide, oxo, or carbamoyl,

As used herein, an "aralkyl" group refers to an alkyl group (e.g., a C₁₋₄ alkyl group) that is substituted with an aryl group. Both "alkyl" and "aryl" have been defined above. An example of an aralkyl group is benzyl.

As used herein, cycloaliphatic encompasses cycloalkyl, cycloalkenyl and cycloalkynyl.

As used herein, a "cycloalkyl" group refers to an aliphatic carbocyclic ring of 3-10 (e.g., 4-8) carbon atoms. Examples of cycloalkyl groups include cyclopropyl, cyclopentyl, cyclohexyl, cycloheptyl, adamantyl, norbornyl, cubyl, octahydro-indenyl, decahydronaphthyl, bicyclo[3.2.1]octyl, bicyclo[2.2.2]octyl, bicyclo[3.3.1] nonyl, and bicyclo[3.2.3]nonyl,. A "cycloalkenyl" group, as used herein, refers to a non-aromatic carbocyclic ring of 3-10 (e.g., 4-8) carbon atoms having one or more double bonds. Examples of cycloalkenyl groups include cyclopentenyl, 1,4-cyclohexa-di-enyl, cycloheptenyl, cyclooctenyl, hexahydro-indenyl, octahydro-naphthyl, bicyclo[2.2.2)octenyl, and bicyclo[3.3.1] nonenyl. A cycloalkyl or cycloalkenyl group can be optionally substituted with one or more substituents such as alkyl (including carboxyalkyl, hydroxyalkyl, and haloalkyl such as trifluoromethyl), alkenyl, alkynyl, cycloalkyl, (cycloalkyl)alkyl, heterocycloalkyl, (heterocycloalkyl)alkyl, aryl, heteroaryl, alkoxy, cycloalkyloxy, heterocycloalkyloxy, aryloxy, heteroaryloxy, aralkyloxy, heteroaralkyloxy, aroyl, heteroaroyl, amino, nitro, carboxy, alkoxycarbonyl, alkylcarbonyloxy, aminocarbonyl, alkylcarbonylamino, cycloalkylcarbonylamino, (cycloalkyl)alkylcarbonylamino, arylcarbonylamino, aralkylcarbonylamino, (heterocycloalkyl)carbonylamino, (heterocycloalkyl)alkylcarbonylamino, heteroarylcarbonylamino, heteroaralkylcarbonylamino, cyano, halo, hydroxy, acyl, mercapto, alkylsulfanyl, sulfoxy, urea, thiourea, sulfamoyl, sulfamide, oxo, or carbamoyl.

As used herein, heterocycloaliphatic means heterocycloalkyl, heterocycloalkenyl and heterocycloalkynyl. As used herein, a "heterocycloalkyl" group refers to a 3- to 10-membered (e.g., 4- to 8-membered) saturated ring structure, in which one or more of the ring atoms is a heteroatom, e.g., N, O, or S. Examples of a heterocycloalkyl group include piperidinyl, piperazinyl, tetrahydropyranyl, tetrahydrofuryl, dioxolanyl, oxazolidinyl, isooxazolidinyl, morpholinyl, octahydro-benzofuryl, octahydro-chromenyl, octahydro-thiochromenyl, octahydro-indolyl, octahydro-pyrindinyl, decahydro-quinolinyl, octahydro-benzo[b]thiophenyl, 2-oxa-bicyclo[2.2.2]octyl, 1-aza-bicyclo[2.2.2]octyl, 3-aza-bicyclo[3.2.1] octyl, anad 2,6-dioxa-tricyclo[3.3.1.03,7]nonyl. A "heterocycloalkenyl" group, as used herein, refers to a 3- to 10-membered (e.g., 4- to 8-membered) non-aromatic ring structure having one or more double bonds, and wherein one or more of the ring atoms is a heteroatom, e.g., N, O, or S. A heterocycloalkyl or heterocycloalkenyl group can be optionally substituted with one or more substituents such as alkyl (including carboxyalkyl, hydroxyalkyl, and haloalkyl such as trifluoromethyl), alkenyl, alkynyl, cycloalkyl, (cyaoalkyl)alkyl, heterocycloalkyl, (heterocycloalkyl)alkyl, aryl, heteroaryl, alkoxy, cycloalkyloxy, heterocycloalkyloxy, aryloxy, heteroaryloxy, aralkyloxy, heteroaralkyloxy, aroyl, heteroaroyl, amino, nitro, carboxy, atkoxycarbonyl, alkylcarbonyloxy, aminocarbonyl, alkylcarbonylamino, cycloalkylcarbonylamino, (cycloalkyl)alkylcarbonylamino, arylcarbonylarmino, aralkylcarbonylamino, (heterocycloalkyl)carbonylamino, (heteracycloalkyl)alkylcarbonylamino, heteroarylcarbonylamino, heteroaralkylcarbonylamino, cyano, halo, hydroxy, acyl, mercapto, alkylsulfanyl, sulfoxy, urea, thiourea, sulfamoyl, sulfamide, oxo, or carbamoyl. In some instances, a susbtituent on the heterocycloalkyl or heterocycloalkenyl itself can be cyclic (which optionally contains one or more hetero atoms) such that the resultant substituted heterocycloalkyl or heterocycloalkenyl is a spiro ring system, e.g., 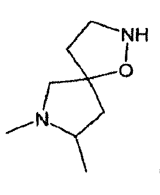 or

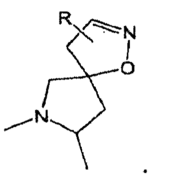

A "heteroaryl" group, as used herein, refers to a monocyclic, bicyclic, or tricyclic ring structure having 5 to 15 ring atoms wherein one or more of the ring atoms is a heteroatom, e.g., N, O, or S and wherein one or more rings of the bicyclic or tricyclic ring structure is aromatic. Some examples of heteroaryl are pyridyl, furyl, pyrrolyl, thienyl, thiazolyl, oxazolyl, imidazolyl, indolyl, 2,3-dihydroindolyl, quinolyl, 1,2-dihydroquinolyl, 1,2,3,4-tetrahydroquinolyl, tetrazolyl, benzofuryl, 2,3-dihydrobenzofuranyl, benzthiazolyl, xanthene, thioxanthene, phenothiazine, dihydroindole, and benzo[1,3]dioxole. A heteroaryl is optionally substituted with one or more substituents such as alkyl (including carboxyalkyl, hydroxyalkyl, and haloalkyl such as trifluoromethyl), alkenyl, alkynyl, cycloalkyl, (cycloalkyl)alkyl, heterocycloalkyl, (heterocycloalkyl)alkyl, aryl, heteroaryl, alkoxy, cycloalkyloxy, heterocycloalkyloxy, aryloxy, heteroaryloxy, aralkyloxy, heteroaralkyloxy, aroyl, heteroaroyl, amino, nitro, carboxy, alkoxycarbonyl, alkylcarbonyloxy, aminocarbonyl, alkylcarbonylamino, cycloalkylcarbonylamino, (cycloalkyl)alkylcarbonylamino, arylcarbonylamino, aralkylcarbonylamino, (heterocycloalkyl)carbonylamino, (heterocycloalkyl)alkylcarbonylamino, heteroarylcarbonylamino, heteroaralkylcarbonylamino, cyano, halo, hydroxy, acyl, mercapto, alkylsulfanyl, sulfoxy, urea, thiourea, sulfamoyl, sulfamide, oxo, or carbamoyl.

A "heteroaralkyl" group, as used herein, refers to an alkyl group (e.g., a C₁₋₄ alkyl group) that is substituted with a heteroaryl group. Both "alkyl" and "heteroaryl" have been defined above.

As used herein, "cyclic moiety" includes cycloalkyl, heterocycloalkyl, cycloalkenyl, heterocycloalkenyl, aryl, or heteroaryl, each of which has been defined previously..

As used herein, an "acyl" group refers to a formyl group or alkyl-C(=O)- where "alkyl" has been defined previously. Acetyl and pivaloyl are examples of acyl groups.

As used herein, a "carbamoyl" group refers to a group having the structure -O-CO-NR^{x}R^{Y} or -NR^{x}-CO-O-R^{z} wherein R^{X} and R^{Y} have been defined above and R^{Z} can be alkyl, aryl, aralkyl, heterocycloalkyl, heteroaryl, or heteroaralkyl.

As used herein, a "carboxy" and a "sulfo" group refer to -COOH and -SO₃H, respectively.

As used herein, an "alkoxy" group refers to an alkyl-O- group where "alkyl" has been defined previously.

As used herein, a "sulfoxy" group refers to -O-SO-R^{x} or -SO-O-R^{x}, where R^{x} has been defined above.

As used herein, a sulfanyl group refers to -S-R^{x}, where R^{x} has been defined above.

As used herein, a sulfinyl group refers to -S(O)-R^{x}, where R^{x} has been defined above.

As used herein, a sulfonyl group refers to -S(O)₂₋R^{x}, where R^{x} has been defined above.

As used herein, a "halogen" or "halo" group refers to fluorine, chlorine, bromine or iodine.

As used herein, a "sulfamoyl" group refers to the structure -S(O)₂-NR^{x}R^{y} or -NR^{x} - S(O)₂-R^{z} wherein R^{x}, R^{Y}, and R^{Z} have been defined above.

As used herein, a "sulfamide" group refers to the structure -NR^{X} -S(O)₂-NR^{Y}R^{Z} wherein R^{x}, R^{Y}, and R^{z} have been defined above.

As used herein, a "urea" group refers to the structure -NR^{x}-CO-NR^{Y}R^{z} and a "thiourea" group refers to the structure -NR^{x}-CS-NR^{y}R^{z}. R^{x}, R^{y}, and R^{Z} have been defined above.

As used herein, a "guanidino" group refers to the structure -N==C(NR^{x}R^{y})N(R^{x}R^{y}) wherein R^{x} and R^{z} have been defined above.

As used herein, the term "amidino group" refers to the structure -C=(NR^{X})N(R^{X}R^{y}) wherein R^{x} and R^{Y} have been defined above.

As used herein, the term "oximino group" refers to the structure -C=N-OR^{x} wherein R^{x} has been defined above.

As used herein, an effective amount is defined as the amount required to confer a therapeutic effect on the treated patient, and is typically determined based on age, surface area, weight, and condition of the patient. The interrelationship of dosages for animals and humans (based on milligrams per meter squared of body surface) is described by Freireich et al., Cancer Chemother. Rep., 50: 219 (1966). Body surface area may be approximately determined from height and weight of the patient. See, e.g., Scientific Tables, Geigy Pharmaceuticals, Ardsley, New York, 537 (1970).

As used herein, "patient" refers to a mammal, including a human.

An antagonist, as used herein, is a molecule that binds to the receptor without activating the receptor. It competes with the endogenous ligand(s) or substrate(s) for binding site(s) on the receptor and, thus inhibits the ability of the receptor to transduce an intracellular signal in response to endogenous ligand binding.

The phrase "optionally substituted" is used interchangeably with the phrase "substituted or unsubstituted." As described herein, compounds of the invention may optionally be substituted with one or more substituents, such as are illustrated generally above, or as exemplified by particular classes, subclasses, and species of the invention. Unless otherwise noted, each of the specific groups for the variables R¹, R², R³, R⁴, and R⁵ in formula (I) may be optionally substituted with one or more substituents described herein. Each substituent of a specific group is further optionally substituted with one to three of halo, cyano, alkoxy, hydroxyl, nitro, haloalkyl, and alkyl. For instance, an alkyl group may be substituted with alkylsulfanyl and the alkylsulfanyl may be optionally substituted with one to three of halo, oxo, cyano, alkoxy, hydroxyl, nitro, haloalkyl, and alkyl. As an additional example, an alkyl may be substituted with a (cycloalkyl)carbonylamino and the cycloalkyl portion of a a (cycloalkyl)carbonylamino may be optionally substituted with one to three of halo, cyano, oxo, alkoxy, hydroxyl, nitro, haloalkyl, and alkyl.

In general, the term "substituted," whether preceded by the term "optionally" or not, refers to the replacement of hydrogen radicals in a given structure with the radical of a specified substituent. Specific substituents are described above in the definitions and below in the description of compounds and examples thereof. Unless otherwise indicated, an optionally substituted group may have a substituent at each substitutable position of the group, and when more than one position in any given structure may be substituted with more than one substituent selected from a specified group, the substituent may be either the same or different at every position. A ring substituent, such as a heterocycloalkyl, may be bound to another ring, such as a cycloalkyl, to form a spiro-bicyclic ring system, e.g., both rings share one common atom. As one of ordinary skill in the art will recognize, combinations of substituents envisioned by this invention are those combinations that result in the formation of stable or chemically feasible compounds.

The phrase "stable or chemically feasible," as used herein, refers to compounds that are not substantially altered when subjected to conditions to allow for their production, detection, and preferably their recovery, purification, and use for one or more of the purposes disclosed herein. In some embodiments, a stable compound or chemically feasible compound is one that is not substantially altered when kept at a temperature of 40° C or less, in the absence of moisture or other chemically reactive conditions, for at least a week.

Unless otherwise stated, structures depicted herein are also meant to include all isomeric (e.g., enantiomeric, diastereomeric, and geometric (or conformational)) forms of the structure; for example, the R and S configurations for each asymmetric center, (Z) and (E) double bond isomers, and (Z) and (E) conformational isomers. Therefore, single stereochemical isomers as well as enantiomeric, diastereomeric, and geometric (or conformational) mixtures of the present compounds are within the scope of the invention. Unless otherwise stated, all tautomeric forms of the compounds of the invention are within the scope of the invention.

Additionally, unless otherwise stated, structures depicted herein are also meant to include compounds that differ only in the presence of one or more isotopically enriched atoms. For example, compounds having the present structures except for the replacement of hydrogen by deuterium or tritium, or the replacement of a carbon by a ¹³C- or ¹⁴C-enriched carbon are within the scope of this invention. Such compounds are useful, for example, as analytical tools or probes in biological assays.

An N-oxide derivative or a pharmaceutically acceptable salt of each of the compounds of formula (I) is also within the scope of this invention. For example, a nitrogen ring atom of the imidazole or pyrazole core ring or a nitrogen-containing heterocyclyl substituent can form an oxide in the presence of a suitable oxidizing agent such as m-chloroperbenzoic acid or H₂O₂.

A compound of formula (I) that is acidic in nature (e.g., having a carboxyl or phenolic hydroxyl group) can form a pharmaceutically acceptable salt such as a sodium, potassium, calcium, or gold salt. Also within the scope of the invention are salts formed with pharmaceutically acceptable amines such as ammonia, alkyl amines, hydroxyalkylamines, and N-methylglycamine. A compound of formula (I) can be treated with an acid to form acid addition salts. Examples of such acids include hydrochloric acid, hydrobromic acid, hydroiodic acid, sulfuric acid, methanesulfonic acid, phosphoric acid, p-bromophenylsulfonic acid, carbonic acid, succinic acid, citric acid, benzoic acid, oxalic acid, malonic acid, salicylic acid, malic acid, fumaric acid, ascorbic acid, maleic acid, acetic acid, and other mineral and organic acids well known to those skilled in the art. The acid addition salts can be prepared by treating a compound of formula (I) in its free base form with a sufficient amount of an acid (e.g., hydrochloric acid) to produce an acid addition salt (e.g., a hydrochloride salt): The acid addition salt can be converted back to its free base form by treating the salt with a suitable dilute aqueous basic solution (e.g., sodium hydroxide, sodium bicarbonate, potassium carbonate, or ammonia). Compounds of formula (I) can also be, for example, in a form of achiral compounds, racemic mixtures, optically active compounds, pure diastereomers, or a mixture of diastereomers.

### B. ABBREVIATIONS

The following abbreviations have the following meanings. If an abbreviation is not defined, it has its generally accepted meaning.

BEMP = 2-tert-butylimino-2- diethylamino- 1,3-dimethylperhydro-1,3,2-diazaphosphorine

Boc = t-butoxycarbonyl

BOP = benzotriazol-1-yloxy-tris (dimethylamino)phosphonium hexafluorophosphate

bd = broad doublet

bs = broad singlet

CDI = carbonyl diimidazole

d = doublet

dd = doublet of doublets

DIC =diisopropylcarbodiimide

DMF = dimethylformamide

DMAP = dimethylaminopyridine

DMSO = dimethylsulfoxide

EDCI = ethyl-1-(3-dimethyaminopropyl)carbodiimide

eq. = equivalents

EtOAc = ethyl acetate

g = grams

HOBT = I-hydroxybenzotriazole

DIPEA = Hunig's base = diisopropylethylamine

L = liter

m = multiplet

M = molar

max = maximum

meq = milliequivalent

mg = milligram

mL = milliliter

mm = millimeter

mmol = millimole

MOC = methoxyoxycarbonyl

N = normal

N/A = not available

ng = nanogram

nm = nanometers

OD = optical density

PEPC = 1-(3-(1-pyrrolidinyl)propyl)-3-ethylcarbodiimide

PP-HOBT= piperidine-piperidine-1-hydroxybenzotrizole

psi = pounds per square inch

Ph = phenyl

q = quartet

quint. = quintet

rpm = rotations per minute

s = singlet

t = triplet

TFA = trifluoroacetic acid

THF = tetrahydrofuran

tlc = thin layer chromatography

µL = microliter

UV = ultra-violet

### II. Compounds of this Invention

Generally, the deuterated compounds of this invention can be synthesized by methods known in the art as for synthesizing their non-deuterated forms, except that a deuterated starting material or a reacting reagent is used during the synthesis process. Examples of applicable methods include those described in U.S. Application No. 60/711,530; WO 02/18369; WO 07/022459; Advanced Organic Chemistry, 2nd Ed., p. 204, J. March, McGraw Hill, New York, NY, 1997; and Synthesis of A: Elemes and Ragnarsosson, J. of Chem. Soc., Perkin 1, 1996,537.

All publications cited herein are incorporated by reference in their entireties.

Compounds of Formula I are prepared using known methods, for example, such as illustrated below in Scheme I.

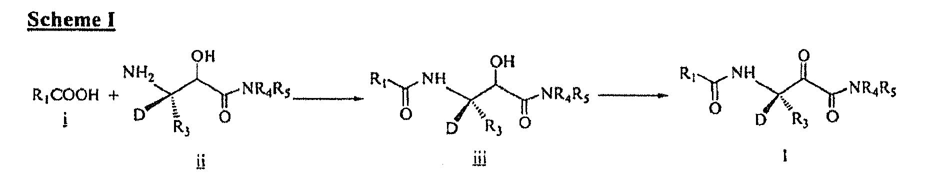

Referring to Scheme I, the acid of Formula i is reacted with a deuterated amino-alcohol-amide of Formula ii in the presence of a condensing reagent such as, for example, EDCI and HOSu to provide the hydroxy-amide of Formula iii. In some embodiments, the percent deuterium (D) enrichment as shown in ii is greater than 10%. In other embodiments the enrichment is from 10% to 99.95%, 40% to 99.95%, 50% to 99.95%, 60% to 99.95%, 80% to 99.95%, 90% to 99.95%, 93% to 99.95%, 97% to 99.95%, or 99-99.95%, or 99.95% or higher. Oxidation of iii with a suitable oxidizing reagent provides the compounds of Formula I. Suitable oxidizing reagents include, for example, Dess-Martin periodinane or TEMPO and sodium hypochlorite.

The deuterated amino-alcohol-amides of Formula ii shown in Scheme 1 can be prepared by using known methods and, for example, as illustrated below in Scheme II.

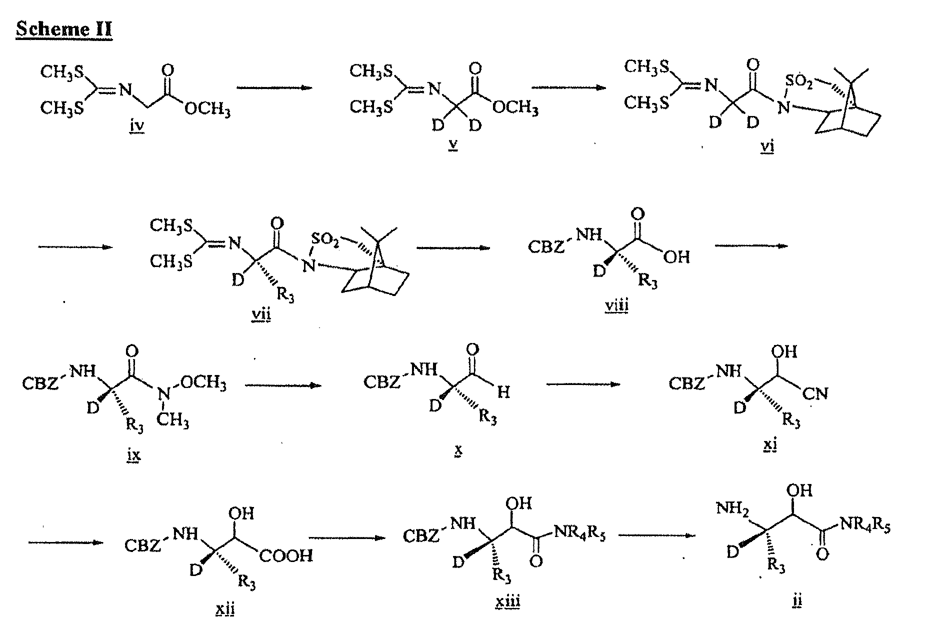

Referring to Scheme II, conversion of the glycine iminic ester iv to the deuterated sultam of Formula vii is conducted according to procedures previously described (Y. Elemes and U. Ragnarsson, J. Chem. Soc., Perkin I, 1996, 6, p.537. Consecutive treatment of compounds of Formula vii with acid and base as previously described (L. Lankiewicz, et. al., J. Chem. Soc., Perkin I, 1994, 17, p.2503 followed by treatment of the intermediate amino acid (not shown) with benzyloxycarbonyl chloride provides the protected deuterated amino acid viii. Reaction of viii with methoxymethylamine in the presence of the condensing reagent CDI provides the Weinreb amide of Formula ix. Reduction of vi with, for example, diisobutylaluminum hydride or lithium aluminum hydride provides the aldehyde x. Using procedures similar to those previously described (see, e.g., WO 02/18369), the aldehyde x is converted to the cyanohydrin xi and thence to the protected hydroxy-amino acid xii. The acid xii is converted to the protected amide xiii which is deprotected to provide the amino-amide ii.

Alternatively, the deuterated amino-amide ii depicted in Scheme I, wherein R₂ is H, may be prepared, e.g., as illustrated in Scheme III.

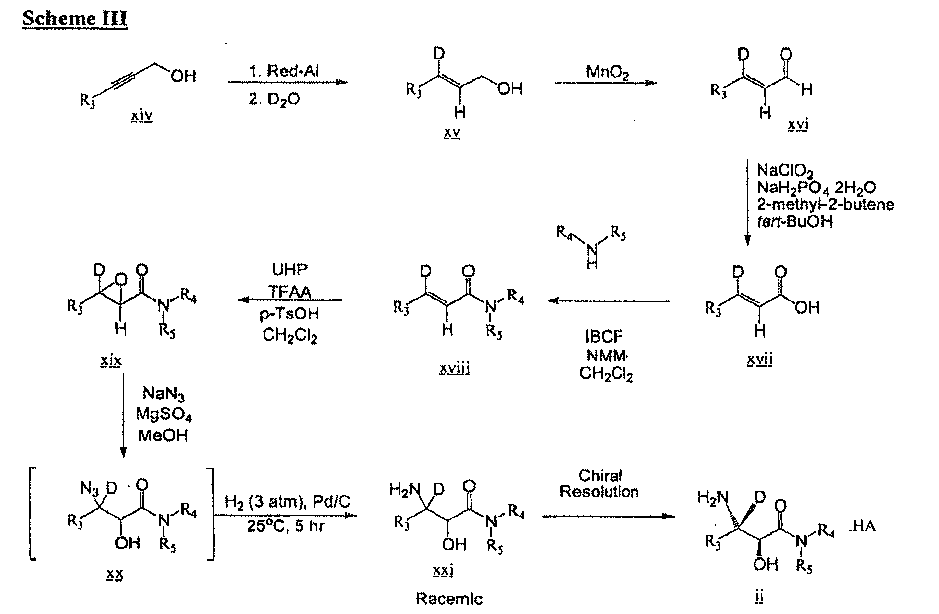

Referring to Scheme III, the propargyl alcohol xiv is reduced with sodium bis(2-methoxyethoxy)aluminumhydride, followed by qenching the reaction mixture with deuterium oxide to provide the deuterated allylic alcohol xv. Oxidation of xv with manganese dioxide provides the aldehyde xvi which is further oxidized to the acid xvii with sodium chlorite (NaClO₂) in the presence of sodium phosphate and 2-methyl-2-butene. Reaction of the acid xvii with isobutylchloroformate (ICBF) in the presence of N-methylmorpholine followed by reaction of the intermediate mixed anhydride with the amine HNR₄R₅ provides the amide xviii. Epoxidation of xviii to provide the epoxide xix may be acheived with urea hydrogen peroxide (UHP) in the presence of trifluoracetic acid and *p*-toluenesulfonic acid. Reaction of xix with sodium azide provides the intermediate azido compound xx which is subsequently reduced to the racemic-aminoalcohol xxi by catalytic hydrogenation in the presence of palladium on carbon. The racemic aminoalcohol xxi may be resolved using known methods such as chiral chromatography, preparation of optically active derivatives or the formation of salts with an optically active acid HA followed by crystallization from an organic solvent. Suitable optically active organic acids for preparing salts include, for example, L-tartaric acid, L-malic acid, (S)-mandelic acid, (1S)-(+)-10-campharsulfonic acid, (-)2,2:4,6-di-O-isopropytidiene-2-keto-L-gulonic acid hydrate, N-acety-L- leucine, deoxycholic acid, (+)-O,O'-dibenzoyl-D-tartaric acid, O,O'-di-(4-toluayl)-D-tartaric acid, S-(+)1,1-binaphtyl-2-2-phosphoric acid, L-lactic acid, D-Gluconic acid, lactobionic acid, dipivaloyl-L-tartaric acid, S-(+)-O-acetylmandelic acid and S-(-)2-(phenylcarbamoyloxy)propionic acid. Examples of suitable organic solvents for recrystallization include dimethylacetamide, ethyl acetate and acetone.

The deuterated compounds thus obtained can be characterized by conventional analytical methods, e.g., NMR and Mass Spectroscope. NMR can be used to determine a compound's structure, while Mass Spectroscopy can be used to determine the amount of deuterium atom in the compound by comparison with its non-deuterated form.

The deuterated compounds of this invention are generally more stable and less inclined to epimerize than their non-deuterated analogs. Thus, they can be used in application where specific steric configuration in the compounds of this invention is desired. For instance, the deuterated compounds of formula (I) may be used to treat or prevent infection caused by HCV or other HCV protease-mediated condition, as they are capable of inhibiting HCV protease. Their inhibitory activity can be measured by traditional enzyme inhibition essays, some of which are described in the publications cited above. See, e.g., Perni, R.B. et al., Antimicrobial Agents and Chemotherapy, 2006 (march), 50 (3): 899-909.

Additionally, the deuterated compounds of formula (I) can be used as a biological tool to study the pharmacological properties of their non-deuterated analogs. Accordingly, these uses are also within the scope of this invention.

### Example 1. Preparation of (1S,3aR,6aS)-2-((S)-2-((S)-2-cyclohexyl-2-(pyrazine-2-carboxamido)acetamido)-3,3-dimethylbutanoyl)-N-((S)-1-(cyclopropylamino)-1,2-dioxo-3-deutero-hexan-3-yl)octahydrocyclopenta[c]pyrrole-1-carboxamide

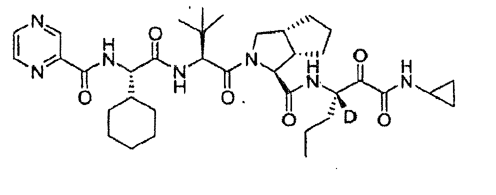

**Step a: Preparation of**

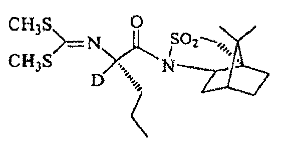

The deuterated sultam (i.e., compound *vi* shown in the scheme below) was prepared by known methods such as those described in Y. Elemes and U. Ragnarsson, J. of Chem. Soc., Perkin 1, 1996, 6, 537; W.Oppolzer, et.al., Helv. Chim. Acta., 1994, 25: 2363, by using the corresponding unsubstituted sultam and propyl iodide.

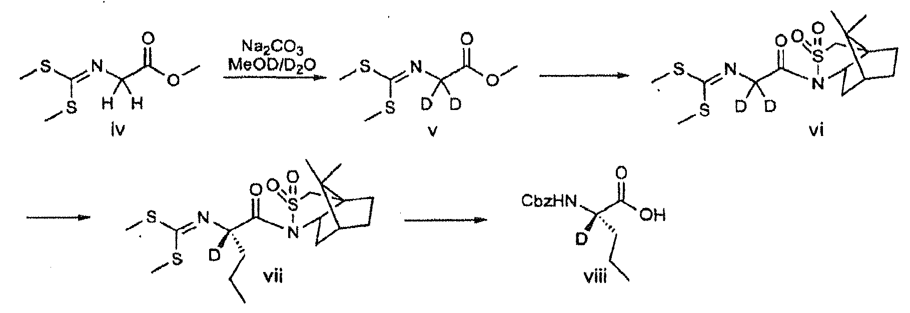

17.32 g of compound *vi* (45.8 mmol) and 229 mL of THF were then charged into a 500 mL round-bottomed-flask with a magnetic stir bar and N₂ inlet. The resulting solution was cooled to -78 °C and n-BuLi (31.5 mL of a 1.6 M solution in hexane, 50.3 mmol) was added with a syringe pump over an hour. The resulting yellow solution was aged for 30 minutes before a solution of HPMA (56 mL) and n-Prl (13.4 mL, 137 mmol) was added to it over 30 minutes. The mixture was allowed to warm to the room temperature over 8 hours and then cooled to -20 °C before D₂O (50 mL) was added to the mixture. The reaction mixture was then extracted with EtOAc (400 mL) and the organic layer was dried over MgSO4 and concentrated to provide 61.3 g of the crude oil. Chromatography on 500 g of silica gel eluting with 2:1 heptane/EtOAc followed by concentration of the rich cut gave 20.35 g of a white solid. The white solid was then recrystallized from EtOH (210 mL) to give 15.39 g of compound vii as a white crystalline solid. The deuterium incorporation was 93% as determined by ¹H NMR.

### Step b: Preparation of (S)-2-(benzytoxycarbomylamino)-2-deuteropentanoic acid, viii

Compound vii (15.39 g, 32.1 mmol) from step a was charged into THF (100 mL) and IN HCl (50 mL). The resulting emulsion was stirred overnight at the room temperature and then concentrated under vacuum to provide a thick oil. The oil was then dissolved in THF (100 mL), and to the solution was added water (25 mL) and LiOH (3.08 g, 128 mmol). This solution was stirred overnight again at the room temperature and then concentrated to remove THF. A hazy light yellow emulsion remained. This was diluted with water (25 mL) and extracted with CH₂Cl₂ (three times, 50 mL each). The aqueous phase was diluted with THF (200 mL) and cooled to 0 °C while stirring rapidly and CBZ-Cl (7.6 mL, 54 mmol) was added dropwise over 15 minutes. After stirring for an hour at 0 °C, the THF solvent was removed *in vacuo* and the residue was acidified by addition of 1N HCl (50 mL). The solution was extracted with EtOAc (3 times, 100 mL each) and the organic phase was dried over Na₂SO₄ and concentrated to provide an oil. The residue was dissolved in EtOAc (25 mL) and heptane (150 mL), seeded and stirred overnight at the room temperature. The solids were collected on a frit, rinsed with heptane (30 mL) and air dried to give 5.65 g (70%) of compound *viii* shown in the scheme above. The deuterium incorporation was 93% as determined by ¹H NMR.

### Step c: Preparation of (S)-benzyl 1-(methoxy(methyl)amino)-1-oxo-2-deuteropentan-2-ylcarbamate

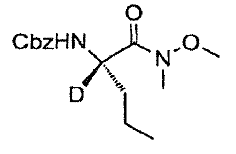

To a flask containing 1.0g of (S)-2-(benzyloxycarbonylamino)-2-deuteropentanoic acid (3.97 mmol) in 20 mL of dichloromethane maintained at 0°C, was added 3.0 eq. of N-methylmorpholine (700 uL), 1.5 eq. of *N,O*-dimethylhydroxylamine hydrochloride (581 mg) and 1.5 eq. of EDCI (1.14 g). The reaction mixture was stirred overnight from 0 °C to the room temperature. The reaction mixture was then diluted in dichloromethane and washed with HCl (1N) and brine. The organic layer was dried over MgSO₄. The crude mixture was purified by flash chromatography (ethyl acetate 15-75% in hexanes) to afford 814 mg of pure amide (title compound). ES+ = 296.1, ES- = 295.2. ¹H NMR spectrum confirmed the structure.

### Step d: Preparation of (S)-benzyl 1-oxo-2-deuteropentan-2-ylcarbamate

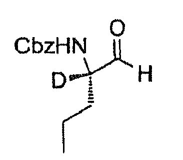

Using procedures described in WO 02/18369, the Cbz-protected amino acid of Step c is converted to the title compound. Specifically, into a flask containing 1.0 eq. of (*S*)-benzyl 1-(methoxy(methyl)amino)-1-oxo-2-deateropentan-2-ylcarbamate (810 mg, 2.75 mmol) in 10 mL of dry THF maintained at 0 °C (in an ice bath) was added slowly 1.7 eq. of a solution of lithium borohydride (1.0M) (4.67 mL). After about 10 minutes, the ice bath was removed and the reaction continue for an hour. The reaction was quenched at 0 °C by adding 5 mL of a solution of KHSO₄ (10%). The solution was then diluted by the addition of 10 mL of HCl (1N). The mixture was stirred for 30 minutes, then extracted 3 times with dichloromethane. The organic phases were combined and washed with a solution of HCl (1 N), water and brine. The organic phase was then dried over MgSO₄ and the volatile evaporated. The aldehyde was used as is in the next step. ES+= 237.1, ES- = 235.2.

### Step e: Preparation of benzyl (3S)-1-(cyclopropylamino)-2-hydroxy-1-oxo-3-deuterohexan-3-ylcarbamate.

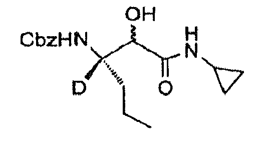

Cyclopropyl isonitrile was prepared according to the scheme shown below. 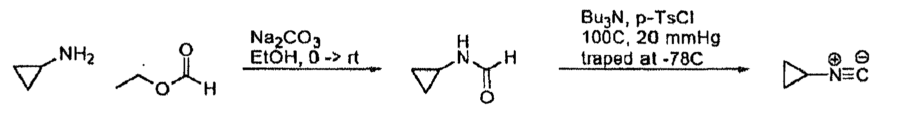 The cyclopropyl isonitrile was then coupled with the aldehyde product of Step d to give the title compound as described in J. E. Semple et al., Org. Lett., 2000, 2(18), p.2769; Lumma W., J. Org. Chem., 1981, 46, 3668". ES+ = 322.1.

### Step f: Preparation of (3S)-3-amino-N-cyclopropyl-3-deutero-2-hydroxyhexanamide

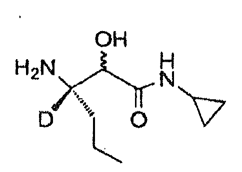

Hydrogenolysis of the Cbz compound of Step e was achieved by using a palladium on carbon catalyst in the presence of hydrogen to give the title compound. Shown in the following schemes are Steps c, d, e, and f.

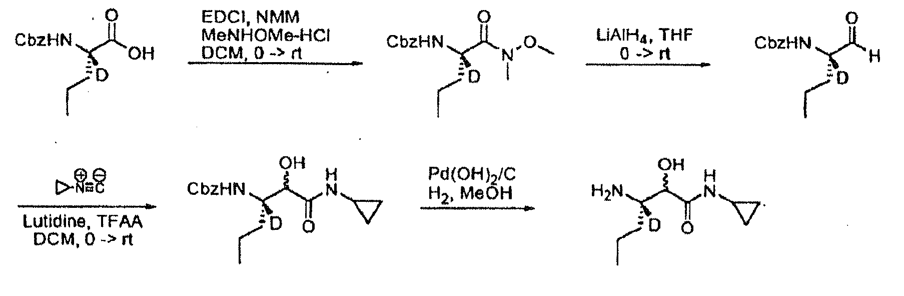

### Step g: Preparation of (1S,3aR,6aS)-2-((S)-2-((S)-2-cyclohexyl-2-(pyrazine-2-carboxamido)acetamido)-3,3-dimethylbutanoyl)-N-((3S)-1-(cyclopropylamino)-3-deutero-2-hydroxy-1-oxohexan-3-yl)octahydrocyclopenta[c]pyrrole-1-carboxamide

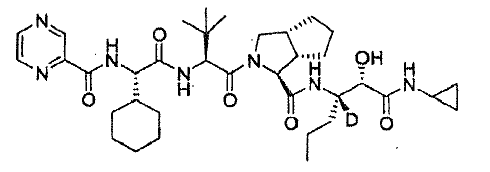

The title compound was prepared from the hydroxy-amino amide product of Step f by condensation with the appropriate acid in the presence of a coupling reagent such as, e.g., EDCI and HOSu. Specifically, in a flask containing 1.2 eq. of (1*S*,3a*R*,6a*S*)-2-((*S*)-2-((*S*)-2-cyclohexyl-2-(pyrazine-2-carboxamido)acetamido)-3,3-dimethylbutanoyl)octahydrocyclopenta[*c*]pyrrole-1-carbaxylic acid (1.59g) in 20 mL of DMF, was added 2.5 eq. of diisopropylamine (980 uL), 1.2 eq. N-hydroxybenzotriazole hydrate (411 mg) and 1.3 eq. of EDCI (558 mg). After 15 minutes of stirring at the room temperature, 1.0 eq. of (35)-3-amino-N-cylopropyl-3-deutero-2-hydroxyhexanamide hydrochloride (500 mg) was added to the mixture. After another 24 hours, the reaction mixture was diluted into 400 mL of ethyl acetate. The organic phase of the mixture was washed with HCl (1N), water, saturated sodium bicarbonate solution, brine, and then dried over MgSO₄. The crude product was purified by chromatography on silica (ethyl acetate 70-100% in Hexanes) to give 1.31 g of the tile compound as a white solid. ES+ = 683.6, ES- = 682.2. The NMR ¹H confirmed the structure.

### Step h: Preparation of (1S,3aR,6aS)-((S)-2-cyclohexyl-2-(pyrazine-2-carboxido)acetamido)-acetamido)-3,3-dimethylbuanoyl)-N-((S)-1-(cyclopropylamino)-1,2-dioxo-3-deutero-hexan-3-yl)octahydropenta[c]pyrrole-1-carbonate

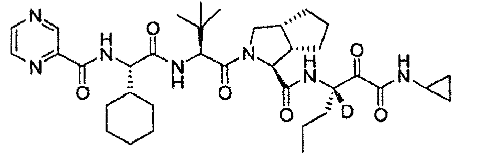

The title compound was prepared by oxidation of the product of Step g with a suitable oxidizing reagent such as Dess Martin periodinane or TEMPO and sodium hypochlorite. Specifically, in a flask containing 1.31g of (1*S*,3a*R*,6a*S*)-2-((*S*)-2-((*S*)-2-cyclohexyl-2-(pyrazine-2-carboxamido)acetamido)-3,3-dimethylbutanoyl)-*N*-((3*S*)-1-(cyclopropylamino)-3-deutero-2-hydroxy-1-oxohexan-3-yl)octahydrocyclopenta[*c*]pyrrole-1-carboxamide in 40 mL of dichloromethane was added at room temperature 1.06g of Dess Martin periodinane. After 2 hours of stirring, 50 mL of sodium bisulfite (1N) was added, and the mixture was stirred for 30 minutes. The 2 phases were separated, the organic was washed with water twice, brine and dried over Na2SO4. The crude product was purified by chromatography on silica (ethyl acetate 20-100% in Hexanes) to give 1.07g of the title compound as a white solid. ES+ = 681.5, ES- = 680.0. The ¹H NMR spectrum confirmed the structure.

Deuterium incorporation was determined by MS to be 93%. The diastereoisome ratio was determined by chiral HPLC normal phase and was higher than 99% d.e.

The following scheme shows the reactions of both Steps g and h.

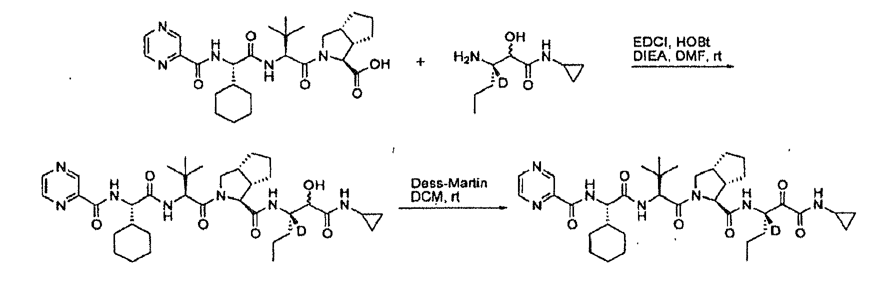

### Example 2: Preparation of (2S,3S)-3-amino-3-deutero-N-cyclopropyl-2-hydroxyhexanamide hydrochloride

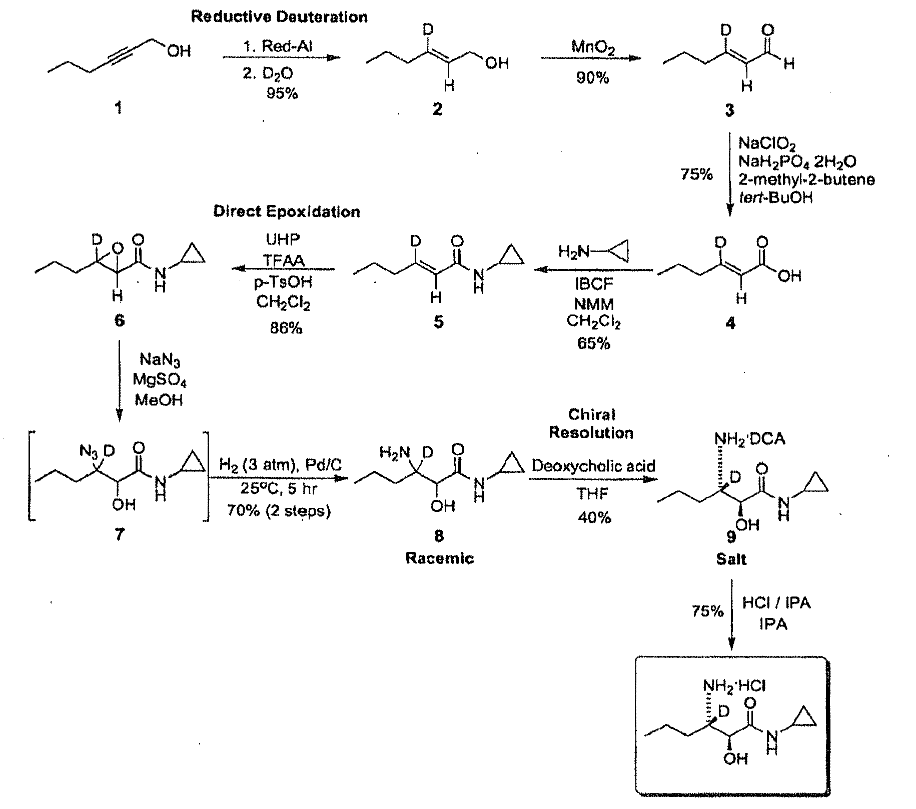

The scheme shown above illustrate the total synthesis of the title compound. Each step is desrcibed in detail as follows.

### Step 1: Preparation of 3-deutero-(E)-hex-2-en-1-ol

To a three-neck 250 mL round bottom flask equipped with mechanical stirrer and reflux condenser was charged 2-hexyn-1-ol (10 g, 0.1 mole) and THF (100 mL, 10 vol). The resulting mixture was cooled to 0±5 °C and then Red-Al (65% in Toluene, 32 mL, 1.6 eq) was added slowly under a nitrogen atmosphere between 0 °C and 20 °C. The resulting mixture was allowed to be warmed up to 25 °C and stirred for 5 hours. The reaction mixture was cooled down to -5±5 °C and D₂O (8.2 g, 4 eq.) was added drop wise between 0 °C and 15°C. To the resulting mixture was charged IPAC (50 mL, 5 vol) and saturated NH₄Cl solution (50 mL, 5 vol.). After stirring the mixture for 10 min, the white solid formed was filtered out. The organic layer from the filtrate was separated and the aqueous layer was extracted with IPAC(30 mL, 3 vol). The organic layers were combined and washed with water (30 mL, 3 vol) and dried over MgSO₄ and concentrated to afford 9.8 g of the product (compound 2) as a colorless oil. The crude product 2 was used for the next step without further purification.
¹H NMR (500 MHz, CDCl₃) δ 5.66 (t, 1H, J=5.0 Hz), 4.12 (d, 2H, J = 5.0 Hz), 2.04 (t, 2H, J=5.0 Hz), 1.38∼1.46 (m, 2H), 0.93 (t, 3H, J=5.0 Hz)

### Step2: Preparation of 3-deutero-(E)-hex-2-enal

To a three-neck 250 mL round bottom flask equipped with mechanical stirrer containing 3-deutero-2-hexenol (10 g, 0.1 mole) in CH₂Cl₂ (150 mL, 15 vol) was charged activated MnO₂ (87 g, 10 eq) at room temperature. After vigorous stirring for 1 hour, another portion of MnO₂ (16 g, 2 eq) was added and the shaking was continued for 4 hours. The reaction solution was filtered through a pad of celite. The solvent was removed in vacuo (25 °C, 100 mmHg) to give 8.8 g of the crude aldehyde product (compound 3) as a pale yellowish oil. The crude product was used for the next step without further purification.
¹H NMR (500 MHz, CDCl₃) δ 9.54 (d, 1 H, J=10.0 Hz), 6.14(s, 1H), 2.34 (m, 2H), 1.55∼1.60 (m, 2H), 1.00 (t, 3H, J=5.0 Hz)

### Step 3: Preparation of 3-deutero-(E)-hex-2-enoic acid

To a three-neck 500 mL round bottom flask equipped with mechanical stirrer and reflux condenser was charged 3-deutero-2-Hexen-1-al (10 g, 0.1 mole), *tert*-BuOH (90 mL, 9 vol), and 2-methyl-2-butene (30 mL, 3 vol). The resulting solution was added with a freshly prepared aqueous NaClO2 (27.4 g, 3 eq) and NaH₂PO₄ (62.9 g, 4 eq) in water (200 mL) over 30 minutes. The reaction mixture was stirred at room temperature for 2 hours. The reaction solution was cooled down to 0 °C and was added with saturated Na₂SO₃ aqueous solution until the reaction color becomes colorless. The stirring was stopped and the organic layer was separated and the aqueous layer was extracted with EtOAc (3 vol × 3). The organic layers were combined and concentrated in vacuo until the total volume becomes 3 vol. The resulting solution was extracted with IN NaOH (3 vol × 3) and the remaining organic layer was discarded. The combined aqueous solution was acidified with 6 N HCl until the pH became 1.0. The solution was extracted with CH₂Cl₂ (3 vol × 5). The combined organic layer were dried over MgSO₄ and concentrated to afford 8.7 g of the product (compound 4) as a white solid.
¹H NMR (500 MHz, CDCl3) δ 5.84 (s, 1H), 2.23 (t, 2H, J=5.0 Hz), 1.51∼1.55 (m, 2H), 0.98 (t, 3H, J=5.0 Hz)

### Step 4: Preparation of 3-deutero-(E)-N-cyclopropylhex-2-enamide

To a three-neck 250 mL round bottom flask equipped with mechanical stirrer and reflux condenser was charged 2-Hexenoic acid-3d (10 g, 0.09 mole), IBCF (13 g, 1.1 eq) in CH₂Cl₂ (100 mL, 10 vol). The resulting solution was cooled down to 0 °C and NMM (13.2 g, 1.5 eq) was added slowly by controlling the temperature between 0 and 20 °C. Then, the mixture was allowed to be warmed up to room temperature and stirred for 1 hour. To the resulting solution was added cyclopropyl amine (5.9 g, 1.2 eq) and the solution was stirred for 2 hours. The reaction mixture was washed with 1N NaOH (3 vol × 2), 1N HCl (3 vol x 2), and brine solution (3 vol), and water (3 vol). The organic layer was dried over MgSO₄ and concentrated to afford the crude product as oil. The crude product was dissolved with heptane (5 vol) and cooled down to -78°C with shirring. The precipitated solid was filter and dried to afford 8.7 g of the product (compound 5) as a white solid.
¹H NMR (500 MHz, DMSO) δ 7.92 (s, 1H), 5.78 (s, 1 H), 2.66∼2.68 (m, 1H), 2.08 (t, 2H, J=5.0 Hz), 1.38∼1.42 (m, 2H), 0.87 (t, 3H, J=5.0 Hz), 0.63 (t, 2H, J=3.0 Hz), 0.40 (t, 2H, J=3.0 Hz)

### Step 5: Preparation of 3-deutero-N-cyclapropyl-3-propyloxirane-2-carbaxamide

To a three-neck 250 mL round bottom flask equipped with mechanical stirrer and containing (E)-N-cyclapropylhex-2-enamide-3d (i.e., product from Step 4) (10 g, 0.06mole), urea hydrogen peroxide (25g, 4 eq), and p-TsOH (12.3 g, 1 eq) in CH₂Cl₂ (100 mL, 10 vol) at 0 °C was added trifluoroacetic anhydride (40.9 g, 3 eq) in CH₂Cl₂ (50 mL, 5 vol) over 30 minutes. The reaction mixture was heated to 40±5 °C and stirred for 3 hours. After cooling to 0°C, the reaction mixture was quenched by adding 6 N NaOH (100 mL, 10 vol) slowly and stirring for 30 minutes. The organic layer was separated and washed with brine (5 vol) and water (5 vol). The washed organic layer was dried over MgSO₄ and solvent evaporated to afford 9.7 g of the epoxide product (i.e., compound 6) as pale yellow oil. The crude product was used for the next step without further purification.
¹H NMR (500 MHz, DMSO) δ 8.01 (s, 1H), 3.09 (s, 1H), 2.63∼2.65 (m, 1H), 1.39∼1.54 (m, 4H), 0.91 (t, 3H, J=5.0 Hz), 0.60 (t, 2H, J=3.0 Hz), 0.45 (t, 2H, J=3.0 Hz)

### Step 6: Preparation of 3-azido-3-deutero-N-cyclopropyl-2-hydroxyhexanamide

To a three necked 250 mL round bottom flask equipped with mechanical stirrer and reflux condenser containing the epoxide-3d 6 (10 g, 0.06 mole) and anhydrous magnesium sulfate (14.1 g, 2.0 eq) in MeOH (100 mL, 10 vol) was added sodium azide (15.3 g, 4.0 eq) in one portion. The resulting mixture was heated to 65±5 °C and stirred for 5 hours. The reaction mixture was cooled to the room temperature and IPAC (100mL, 10vol) was added and the mixture was stirred for another 10 minutes. The mixture was filtered through a pad of Celite^{®} to remove insoluble salts and the resulting clear solution was concentrated to 3 vol. To the resulting solution was added IPAC (170mL, 17vol) and the mixture was stirred for another 10 minutes. Again, the solution was filtered through a pad of Celite^{®} to afford the product, the azide-3d (compound 7), as a clear solution in IPAC (about 200 mL), which was used for the next step without further purification.
¹H NMR (500 MHz, DMSO) δ 7.91 (s, 1H), 6.00 (d, 1H, J=5.0 Hz), 4.03 (d, 1H, J=5.0 Hz), 2.66∼2.67 (m, 1H), 1.30∼1.58 (m, 4H), 0.88 (t, 3H, J=5.0 Hz), 0.60 (t, 2H, J=3.0 Hz), 0.48 (t, 2H, J=3.0 Hz)

### Step 7: Preparation of 3-amina-3-deutero-N-cyclopropyl-2-hydraxyhexanamide

To a 500 mL of autoclave hydrogenation reactor equipped with mechanical stirrer containing the azide-3d 7 (200 mL, 0.05 mole) in IPAC obtained in the previous step in a hydrogenation reactor was charged Pd/C (10% Pd, water 50%, 0.8 g). The solution was charged with nitrogen (1.0 atm) and released three times and then charged with hydrogen (3.0 atm) and released three times. The resulting solution was charged with hydrogen (3 atm) and stirred for 5 hours. After releasing the hydrogen gas, the solution was purged with nitrogen for 5 minutes. To the resulting solution was added MeOH(30ml, 3 vol) and the reaction mixture was heated to 50 ± 5 °C. The reaction mixture was filtered through a pad of celite to afford a clear solution. The product was isolated by concentrating the solution at 20 ± 5°C until 3 vol of the solution remained. The solid was collected by filtration, washed (IPAC, 3 vol), and dried to give 7.7 g of the title compound (compound 8) as a white crystalline solid.
¹H NMR (500 MHz, DMSO) δ 7.70 (s, 1H), 5.31(s, 2H), 3.68 (s, 1H), 2.64∼2.66 (m, 1H), 1.10∼1.50 (m, 4H), 0.82 (t, 3H, J=5.0 Hz), 0.59 (t, 3H, J=3.0 Hz), 0.45 (t, 3H, J=3.0 Hz)

### Step 8: Preparation of (2S,3S)-3-amino-3-deutero-N-eyelopropyl-2-hydroxyhexanamide deoxycholate

Deoxycholic acid (15.7 g, 0.75 eq.) was charged to a three-neck 250 mL round bottom flask equipped with mechanical stirrer and containing the racemic (2S,3S)-3-amino-3-deutero-N-eyelopropyl-2-hydroxyhexanamide of *step* 7 (10 g, 0.05 mole) in THF (100 mL, 10 v). The reaction mixture was heated to 65±5 °C and stirred for 1 hour. The resulting homogeneous mixture was cooled to 23+2 °C over 1 hour, and left at the same temperature range for 1 hour. The precipitated solids were collected by filtration, washed with THF (50 mL, 5 vol), and dried to give 12.4 g of the salt compound (compound 9) as a white solid. The product has an enatiomeric ratio(ER) of 2:98.

### Step 9: Preparation of (2S,3S)-3-amino-3-deutero-N-cyclopropyl-2-hydroxyhexanamide hydrochloride

To a three-neck 250 mL round bottom flask equipped with mechanical stirrer was charged the dihydrocholate salt (from *step 8*) and 2-propanol (62 mL, 5 vol). The solution was heated to 75±5 °C and 5 to 6 N HCl solution in IPA (12 mL, 3 eq.) was added slowly with vigorous stirring. The resulting solution was stirred at the same temperature for I hour and then cooled down to 23±2 °C. The reaction mixture was maintained at the same temperature for 1 hour. The precipitated solids were collected by filtration, washed with 2-propanol (36 mL, 3 vol), dried to give 3.0 g of the title compound (enantiomeric ratio = 0:100) as a white solid. The deuterium incorporation was higher than 99% as determined by MS and ¹H NMR.
¹H NMR (500 MHz, DMSO) δ 8.07 (s, 1H), 7.97 (s, 3H), 6.25 (d, 1H, J=5.0 Hz), 4.16 (d, 1H, J=5.0 Hz), 2.67∼2.70 (m, 1H), 1.33∼1.46(m, 4H), 0.84 (t, 3H, J=5.0 Hz), 0.61 (t, 3H, J=3.0 Hz), 0.53 (t, 3H, J=3.0 Hz).

### Example 3. Assay for Measuring Epimerization Rate

The deuterated compounds of this invention undergo slow epimerization as follows:

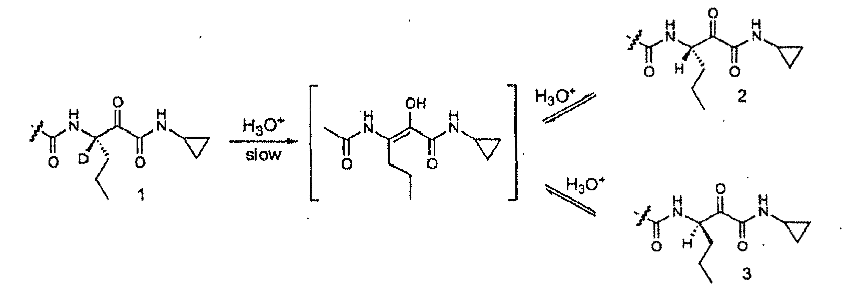

The epimerization rate was measured according to the following assay. Specifically, 100 µL medium (buffer, rat plasma, dog plasma, or human plasma) was added into a 96-well deep plate. To the plasma was then added 10 µL acetonitrile solution containing a test compound (1*S*,3a*R*,6a*S*)-2-((*S*)-2-((*S*)-2-cyclohexyl-2-(pyrazine-2-carboxamido)acetamido)-3,3-dimethylbutanoyl)-*N*-((*S*)-1-(cyclopropylamino)-1,2-dioxo-3-deutero-hexan-3-yl)octahydracyclopenta[*c*]pyrrole-1-carboxamide (at 1 uM or 10 uM) and 1200 µL ethyl acetate into the 96 deep-well plate (2mL) by using a TomTec liquid handling workstation (Hamden, CT, USA). The plate was then covered tightly and shaken with a vortex for 20 minutes before it was centrifuged at 3000 rpm for 10 minutes. After centrifuge, 900 µL of the supernatant was transferred to a new V-shape 96 deep-well plate using TomTec, and then dried under nitrogen gas (flow rate of 60 L/min) at 25 °C for about 30 minutes. The residue was reconstituted with 100 µL ethyl acetate, and the solution was again transferred into the glass inserts in the 96-well plate. 20 uL of the reconstituted solution was injected into LC-MS/MS to determine the amount of the epimers. The LC-MS/MS spectrometer used a ChiralPak AD Column (4.6×150 mm, 10 µm), a mixture of isopropanol and n-heptane (10:90, 50:50, or 90:10) as the mobile phase, and isopropanol as the washing solvent. Also used in the MS spectrometer was a deuterated analog of the test compound containing 11 deuterium atoms in the cyclohexyl group (C₃₆H₄₂D₁₁N₇O₆, MW 690.47).

The test compound had a mass (M+H, m/z) of 681.36, while its non-deuterated analogs (with the same or different chiral configurations at the deuterated carbon center) had a mass (M+H, m/z) of 680.36. Their LC-MS/MS spectra showed a fragment of 323.30 (with deuterium) and 322.30 (non-deuterated).

At both concentrations (1 uM and 10 uM) and in the same medium (i.e., a buffer, rate plasma, dog plasma, and human plasma), the test deuterated compound of formula (I) showed a slower epimerization rate than its non-deuterated form buffer, rat plasma, and dog plasma; and a much slower epimerization rate in human plasma. For instance, in human plasma and at 1 uM or 10, uM, the deuterated compound epimerized for about 30% in 180 minutes, whereas the non-deuterated form epimerized for almost 40%. In addition, in human plasma, the deuterated compound epimerized at a linear rate for 180 minutes, while the non-deuterated form showed an exponential rate of epimerization in the first 60 minutes before it leveled off.

### Example 4: Assay for Determining IC₅₀ in HCV Replicon Cells

(1*S*,3a*R*,6a*S*-2-((*S*)-2-((*S*)-2-cyclohexyl-2-(pyrazine-2-carboxamido)acetamido)-3,3-dimethylbutanoyl)-*N*-((*S*)-1-(cyclopropylamino)-1,2-dioxo-3-deutero-hexan-3-yl)octahydrocyclopenta[c]pyrrole-1-carboxamide and (5*S*,8*S*)-3-(5-chloro-2,4-dimethoxyphenyl)-7-((*S*)-2-(2-cyclohexylacetamido)-3,3-dimethylbutanoyl)-*N*-((*S*)-1-(cyclopropylwnino)-1,2-dioxo-3-deuterohexan-3-yl)-1-oxa-2,7-diazaspiro[4.4]non-2-ene-8-carboxamide were used in this assay as described in Lin, C. et al., J. Biol. Chem., 2004, 279: 17508-17514; Lin, K. et al., Antimicrob. Agents Chemother., 2004, 48:4784―4792.

Huh-7 cells harboring an autonomously replicating, subgenomic HCV replicon of the Con1 strain were maintained in Dulbecco's modified Eagle's medium (DMEM), 10% heat-inactivated fetal bovine serum (FBS), 2 mM L-glutamine, and nonessential amino acids (JRH Biosciences, Lenexa, KS), plus 0.25 mg/ml G418 (Invitrogen, Carlsbad, CA). The subgenomic HCV replicon also encodes a neomycin phosphotransferase, which allows selective growth of HCV replicon-containing Huh-7 cells over HCV replicon-negative Huh-7 cells in the presence of G418. The concentrations of the test compound at which the HCV RNA level in the replicon cells is reduced by 50% (IC₅₀) or by 90% (IC₉₀) or the cell viability is reduced by 50% (CC₅₀), were determined in HCV Con1 subgenomic replicon cells (19) using 4-parameter curve fitting (SoftMax Pro). The replicon cells were incubated with the test compound diluted in DMEM containing 2% FBS and 0.5% DMSO (without G418) at 37 °C. Total cellular RNA was extracted using an RNeasy-96 kit (QIAGEN, Valencia, CA), and the copy number of the HCV RNA was determined in a quantitative, real-time, multiplex reverse transcription-PCR (QRT-PCR, or Taqman) assay. The cytotoxicity of compounds in the HCV replicon cells was measured under the same experimental settings using the tetrazolium-based cell viability assay.

The results show that both test compounds have a Ki of less than 50 nM, and an IC₅₀ (over 5 days) of less than 10.0 uM.

### OTHER EMBODIMENTS

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.
The present invention further comprises the aspects defined in the following clauses (which form part of the present description but are not considered as claims in accordance with decision J 15/88 of the Legal Board of Appeal of the European Patent Office):
1. A deuterium-enriched α-ketoamido compound of the formula 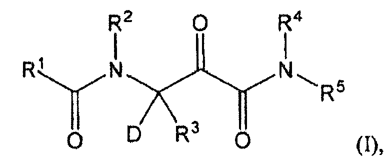 wherein:
   D denotes a deuterium atom; 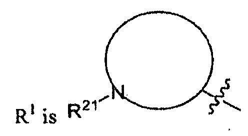 in which 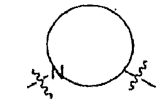 is an optionally substituted monocyclic azaheterocyclyl or optionally substituted multicyclic azaheterocyclyl, or optionally substituted multicyclic azaheterocyclenyl wherein the unsaturatation is in the ring distal to the ring bearing the R²¹ moiety and to which the -C(O)-N(R²)-CDR³-C(O)-C(O)-NR⁴R⁵ moiety is attached;
   R²¹ is Q³-W³-Q²-W²-Q¹; wherein
   Each of W² and W³ is independently a bond, -CO-, -CS-, -C(O)N(Q⁴)-, -CO₂-, -O-, -N(Q⁴)-C(O)-N(Q⁴)-, -N(Q⁴)-C(S)-N(Q⁴)-, -OC(O)NQ⁴-, -S-, -SO-, -SO₂-, - N(Q⁴)-, -N(Q⁴)SO₂-, -N(Q⁴)SO₂N(Q⁴)-, and hydrogen when any of W² and W³ is the terminal group;
   Each of Q¹, Q², and Q³ is independently a bond, an optionally substituted aliphatic, an optionally substituted heteroaliphatic, an optionally substituted cycloaliphatic, an optionally substituted aryl, an optionally substituted heteroaryl, an optionally substituted aralkyl, or an optionally substituted heteroaralkyl; or hydrogen when any of Q³, Q², or Q¹ is the terminal group, provided that Q² is not a bond when both W³ and W² are present; and
   Each of R², R³, and R⁴, independently, is H or a C₁₋₆ alkyl; and
   R⁵ is H, alkyl, cycloalkyl, aryl optionally substituted with 1-4 alkyl groups, alkylaryl, aryl, amino optionally substituted with 1 or 2 alkyl groups.
2. The compound of clause 1, wherein R²¹ is 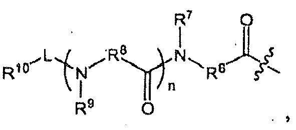 in which
   each of R⁶ and R⁸ is independently
   a bond; or
   optionally substituted (1,1- or 1,2-)cycloalkylene; or
   optionally substituted (1,1- or 1,2-)heterocyclylene; or
   methylene or ethylene, substituted with one substituent selected from the group consisting of an optionally substituted aliphatic group, an optionally substituted cyclic group and an optionally substituted aromatic group, and wherein the methylene or ethylene is further optionally substituted with an aliphatic group substituent;
   each of R⁷, R⁹, and R¹¹ is independently hydrogen or optionally substituted aliphatic group;
   R¹⁰ is an optionally substituted aliphatic group, optionally substituted cyclic group or optionally substituted aromatic group;
   L is -C(O)-, -OC(O)-, -NR¹¹C(O)-, -S(O)₂-, -NR¹¹S(O)₂-, or a bond;
   n is 0 or 1.
3. The compound of clause 2, wherein n is 1.
4. The compound of clause 2, wherein R⁶ is methylene substituted with one substituent selected from the group consisting of an optionally substituted aliphatic group, an optionally substituted cyclic group, and an optionally substituted aromatic group.
5. The compound of clause 4, wherein R⁶ is methylene substituted with isobutyl.
6. The compound of clause 2, wherein R⁷ is hydrogen.
7. The compound of clause 2, wherein R⁸ is methylene substituted with one substituent selected from the group consisting of an optionally substituted aliphatic group, an optionally substituted cyclic group, and an optionally substituted aromatic group.
8. The compound of clause 7, wherein R⁸ is methylene substituted with an optionally substituted cyclic group.
9. The compound of clause 8, wherein R⁸ is methylene substituted with cyclohexyl.
10. The compound of clause 2, wherein R⁹ is hydrogen.
11. The compound of clause 2, wherein L is -CO-.
12. The compound of clause 2, wherein R¹⁰ is an optionally substituted aromatic group.
13. The compound of clause 12, wherein R¹⁰ is selected from the group consisting of 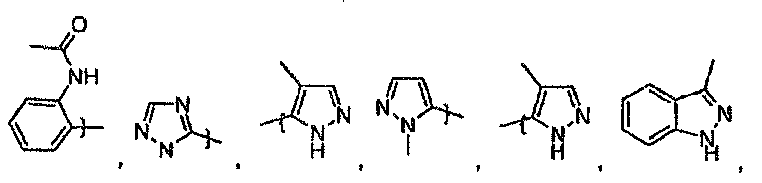 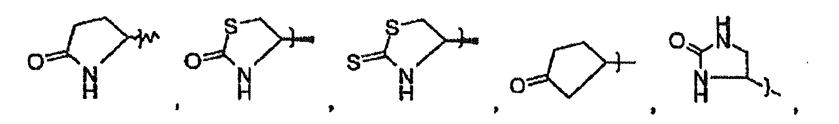 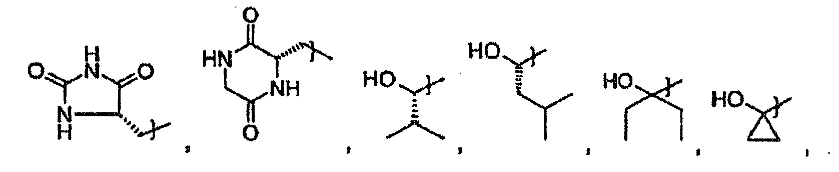 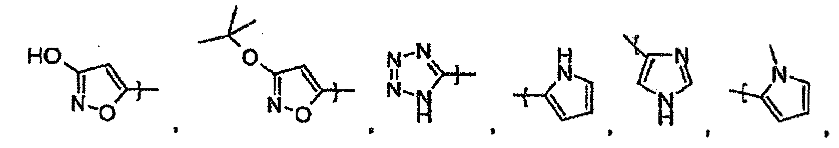 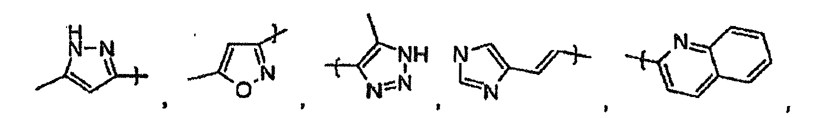 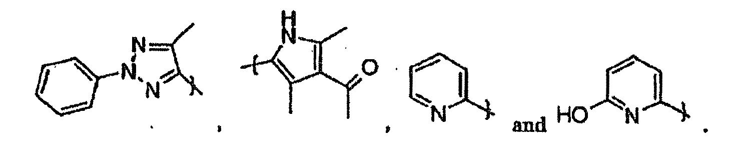
14. The compound of clause 12, wherein R¹⁰ is optionally substituted pyrazinyl.
15. The compound of clause 14, wherein R¹⁰ is 2-pyrazinyl.
16. The compound of clause 2, wherein 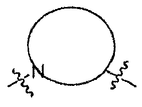 is substituted monocyclic azaheterocyclyl.
17. The compound of clause 16, wherein 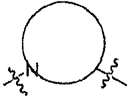 is pyrrolidinyl substituted at C-3 position with heteroaryloxy, wherein the heteroaryl is further optionally substituted with 1-4 halo groups.
18. The compound of clause 16, wherein 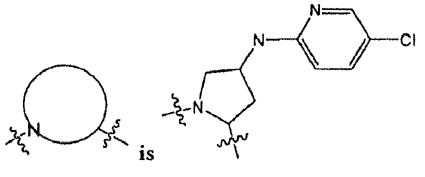 or 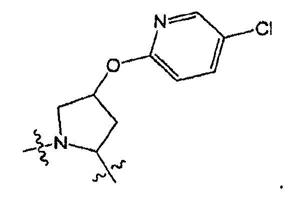
19. The compound of clause 2, wherein 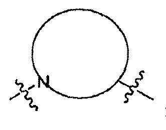 is optionally substituted multicyclic azaheterocyclyl.
20. The compound of clause 19, wherein 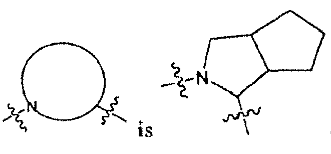 or 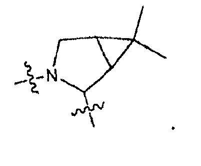
21. The compound of clause 20, wherein 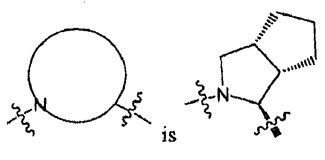 or 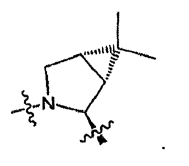
22. The compound of clause 2, wherein R² is hydrogen, each of R⁴ and R⁵ independently is hydrogen or cyclopropyl.
23. The compound of clause 2, wherein R³ is propyl.
24. The compound of clause 2, wherein n is 0.
25. The compound of clause 2, wherein L is -NR¹¹C(O)- and R¹¹ is hydrogen.
26. The compound of clause 2, wherein R¹⁰ is an optionally substituted aliphatic group.
27. The compound of clause 26, wherein R¹⁰ is t-butyl.
28. The compound of clause 2, wherein the compound is 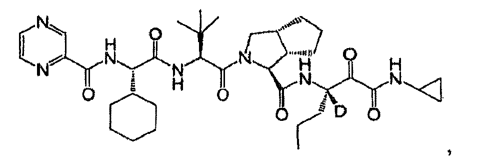 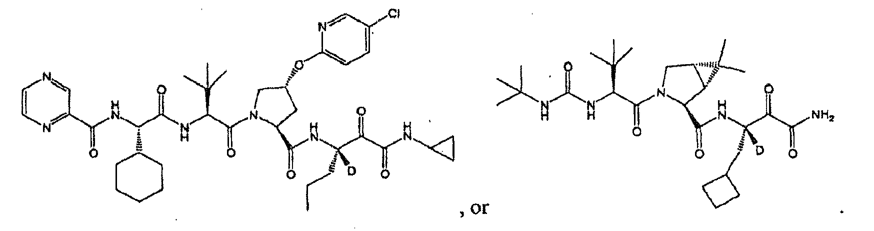
29. The compound of clause 19, wherein 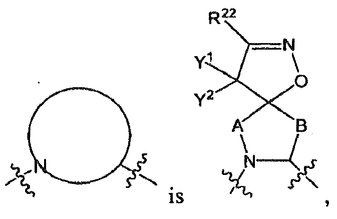 in which
   A is -(CHX¹)ₐ-;
   B is -(CHX²)_{b}-;
   a is 0 to 3;
   b is 0 to 3, provided that a + b is 2 or 3;
   each of X¹ and X² is independently selected from hydrogen, optionally substituted C₁₋₄ aliphatic, and optionally substituted aryl;
   each of Y¹ and Y² is independently hydrogen, optionally substituted aliphatic, optionally substituted aryl, amino, or -OQ⁴; wherein each Q⁴ is independently hydrogen or an optionally substituted aliphatic;
   R²² is an optionally substituted aliphatic, an optionally substituted heteroaliphatic, an optionally substituted cycloaliphatic, an optionally substituted heterocycloaliphatic, an optionally substituted aryl, or an optionally substituted heteroaryl.
30. The compound of clause 29, wherein R²¹ is optionally substituted alkylcarbonyl.
31. The compound of clause 30, wherein R²¹ is aminoalkylcarbonyl, haloalkylcarbonyl, arylalkylcarbonyl, arylalkylcarbonyl, cycloaliphaticalkylcarbonyl, or heterocycloaliphaticalkylcarbonyl, each of which is optionally substituted with 1-3 substituents.
32. The compound of clause31, wherein R²¹ is heterocycloalkyl-oxycarbonylamino-alkylcarbonyl, heteroaryl-carbonylamino-alkyl-carbonylamino-alkyl-carbonyl, bicycloaryl-sulfonylamino-alkylearbonyl, aryl-alkoxy-carbonylamino-alkyl-carbonyl, alkyl-carbohylamino-alkyl-carbonyl, aliphatic-oxycarbonylamino-alkyl-carbonyl, cycloaliphatic-alkyl-aminocarbonylamino-alkyl-carbonyl, cycloaliphatic-alkyl-carbonylamino-alkyl-carbonyl, heteroaryl-carbonylamino-alkyl-carbonylamino-alkyl-carbonyl, alkyl-aminocarbonylamino-alkyl-carbonyl, or bicycloaryl-aminocarbonylamino-alkyl-carbonyl, each of which is optionally substituted with 1-3 substituents.
33. The compound of clause 29, wherein R²¹ is 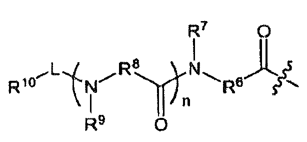 in which
   each of R⁶ and R⁸ is independently
   a bond; or
   optionally substituted (1,1- or 1,2-)cycloalkylene; or
   optionally substituted (1,1- or 1,2-)heterocyclylene; or
   methylene or ethylene, substituted with one substituent selected from the group consisting of an optionally substituted aliphatic group, an optionally substituted cyclic group and an optionally substituted aromatic group, and wherein the methylene or ethylene is further optionally substituted with an aliphatic group substituent;
   each of R⁷, R⁹, and R¹¹ is independently hydrogen or optionally substituted aliphatic group;
   R¹⁰ is an optionally substituted aliphatic group, optionally substituted cyclic group or optionally substituted aromatic group;
   L is -C(O)-, -OC(O)-, -NR¹¹C(O)-, -S(O)₂-, -NR¹¹(O)₂-, or a bond;
   n is 0 or 1,
34. The compound of clause 29, wherein R²² is an optionally substituted aliphatic, optionally substituted heteroaliphatic, optionally substituted cycloaliphatic, optionally substituted heterocycloaliphatic, optionally substituted aryl, or optionally substituted heteroaryl.
35. The compound of clause 34, wherein R²² is optionally substituted phenyl, optionally substituted naphthyl, optionally substituted anthracenyl, optionally substituted naphthalene, or optionally substituted anthracene.
36. The compound of clause 29, wherein each of X¹, X², Y¹, and Y² is hydrogen, each of a and b is 1.
37. The compound of clause 36, wherein R²² is an optionally substituted aliphatic, optionally substituted heteroaliphatic, optionally substituted cycloaliphatic, optionally substituted heterocycloaliphatic, optionally substituted aryl, or optionally substituted heteroaryl.
38. The compound of clause 37, wherein R²² is an optionally substituted phenyl, optionally substituted naphthyl, optionally substituted anthracenyl, optionally substituted naphthalene, or optionally substituted anthracene.
39. The compound of clause 38, wherein R²¹ is heterocycloalkyl-oxycarbonylamino-alkylcarbonyl, heteroaryl-carbonylamino-alkyl-carbonylamino-alkyl-carbonyl, bicycloaryl-sulfonylamino-alkylcarbonyl, aryl-alkoxy-carbonylamino-alkyl-carbonyl, alkyl-carbonylamino-alkyl-carbonyl, aliphatic-oxycarbonylamino-alkyl-carbonyl, cycloaliphatic-alkyl-aminocarbonylamino-alkyl-carbonyl, cycloaliphatic-alkyl-carbonylamino-alkyl-carbonyl, heteroaryl-carbonylamino-alkyl-carbonylamino-alkyl-carbonyl, alkyl-aminocarbonylamino-alkyl-carbonyl, or bicycloaryl-aminocarbonylamino-alkyl-carbonyl, each of which is optionally substituted with 1-3 substituents.
40. The compound of clause 29, wherein 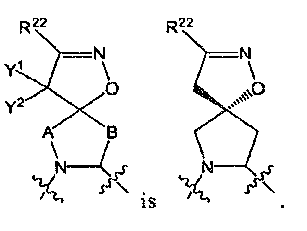
41. The compound of clause 40, wherein the compound is of the structure: 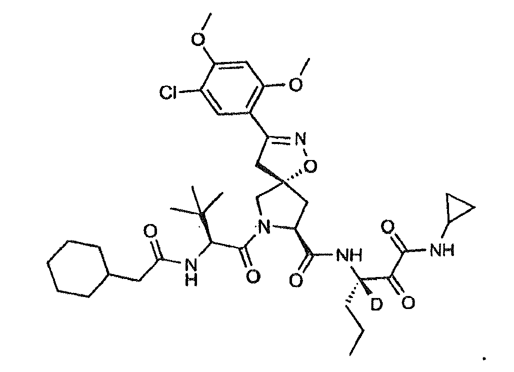
42. The compound of clause 1, wherein the deuterium enrichment is at least 50% in the compound.
43. The compound of clause 42, wherein the deuterium enrichment is at least 80%.
44. The compound of clause 43, wherein the deuterium enrichment is at least 90%.
45. The compound of clause 44, wherein the deuterium enrichment is at least 99%.
46. A pharmaceutical composition comprising a pharmaceutically acceptable carrier and a compound of any of clause 1, 28, 41, 44, or 45.
47. A method for increasing the concentration of the active isomer of a pharmaceutical agent *in vivo,* comprising administering to a patient in need thereof a deuterated isomer of the pharmaceutical agent in an amount sufficient to confer the pharmaceutical effect.
48. The method of clause 47, wherein the deuterated isomer of the pharmaceutical agent is a compound of any of clauses 1, 28, 41, 44, or 45.
49. A method for enhancing the bioavailability of a compound, comprising replacing a hydrogen atom that is bonded to a steric carbon atom in the compound with a deuterium atom.
50. The method of clause 49, wherein the deuterated compound thus obtained is a compound of any of clauses 1, 28, 41, 44, or 45.
51. A method for inhibiting HCV protease, comprising contacting HCV protease with a compound of any of clauses 1, 28, 41, 44, or 45.
52. A method for treating a patient suffering from HCV infection or a condition mediated by HCV protease, comprising administering to the patient a pharmaceutically effective amount of a compound of any of clauses 1, 28, 41, 44, or 45.
53. A process for preparing an optically enriched compound of Formula 1 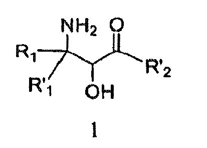 wherein:
   the carbon atoms alpha and beta to the carboxy group are stereocenters;
   R₁ is independently H, optionally substituted aliphatic, optionally substituted cycloaliphatic, optionally substituted arylaliphatic, optionally substituted heteroaliphatic or optionally substituted heteroarylaliphatic;
   R'₁ is deuterium such that the deuterium enrichment is at least 50%;
   R'₂ is -NHR₂ or -OE;
   R₂ is H, optionally substituted aliphatic, optionally substituted cycloaliphatic, optionally substituted arylaliphatic, optionally substituted heteroaliphatic or optionally substituted heteroarylaliphatic; and
   E is C₁₋₆ alkyl or benzyl;
   comprising the steps of :
   a) forming a salt of a compound of Formula 1, and
   b) crystallizing said salt to give a compound of greater than 55% enantiomeric excess.
54. The process of clause 53, wherein R₁ is C₁₋₆ alkyl, and R'₂ is -NHR₂ wherein R₂ is a C₁₋₆ alkyl or C₁₋₆ cycloalkyl.
55. The process of clause 54, wherein R₁ is propyl and R₂ is cyclopropyl.
56. The process of clause 53, further comprising aminating a compound of Formula ii 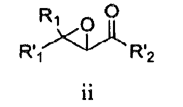 with an aminating reagent to provide a compound of Formula iii 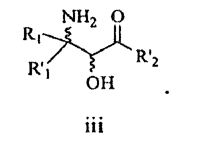
57. The process of clause 56, wherein the aminating reagent is an azide salt and the intermediate azido compound is reduced by hydrogenation.
58. The process of clause 56, further comprising oxidizing an unsaturated compound of Formula i 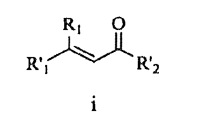 wherein R'₂ is -NHR₂ or -OE, wherein E is C₁₋₅ alkyl or optionally substituted benzyl, with an oxidizing reagent to provide a compound of Formula ii. 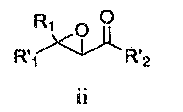
59. The process of clause 58, wherein the oxidizing reagent comprises t-butyl hydroperoxide.
60. The process of clause 59, wherein the oxidizing reagent further a chiral reagent.
61. The process of clause 58, wherein the oxidizing reagent is a mixture of samarium (III) isopropoxide, triphenyl arsine oxide, S-(-)1,1'-bi-2-naphthol and 4 A molecular sieves.
62. The process of clause 58, wherein the oxidizing reagent comprises urea-hydrogen peroxide in the presence of trifluoroacetic anhydride.
63. The process of clause 62, wherein R'₂ is -OE.
64. The process of clause 62, wherein R'₂ is -NHR₂.
65. The process of clause 58, further comprising hydrolyzing the compound of Formula ii to give an acid and then converting the acid to an amide compound of Formula ii wherein R'₂ is -NHR₂.
66. The process of clause 58, further comprising oxidizing a compound of Formula iv 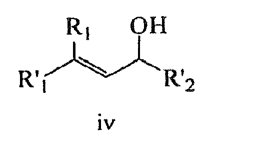 to give the compound of Formula ii.
67. The process of 66, wherein the oxidation is conducted by using manganese dioxide.
68. The process of 66, further comprising reducing a compound of Formula v 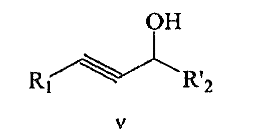 to give the compound of Formula iv.
69. The process of 68, wherein the compound is reduced with Red-A1^{®} and then quenched with deuterium oxide.
70. A process for preparing a compound of Formula 1 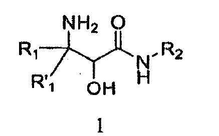 wherein:
   R₁ is H, optionally substituted aliphatic, optionally substituted cycloaliphatic, optionally substituted arylaliphatic, optionally substituted heteroaliphatic or optionally substituted heteroarylaliphatic;
   R'₁ is deuterium,
   R₂ is H, optionally substituted aliphatic, optionally substituted cycloaliphatic, optionally substituted arylaliphatic, optionally substituted heteroaliphatic or optionally substituted heteroarylaliphatic; and
   the compound of Formula 1 has an enantiomeric excess of greater than 55%, comprising the steps of:
      a) oxidation of an unsaturated compound of Formula i 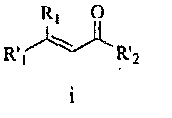 to provide a compound of formula ii 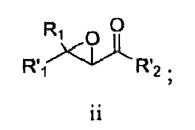
      b) reacting a compound of Formula ii with an aminating reagent to provide a compound of Formula iii 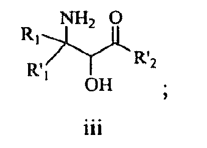
      c) forming a salt of a compound of Formula iii with an optically active organic acid;
      d) crystallizing said salt to give a compound of greater than 55% enantiomeric excess.
71. The process of clause 70, wherein the compound of Formula 1 is (2S,3S)-3-amino-3-deutero-N-cyclopropyl-2-hydroxyhexanamide.
72. The process of clause 71, wherein the organic acid is L-tartaric acid or deoxycholic acid.

## Claims

1. A deuterium-enriched α-ketoamido compound of the formula 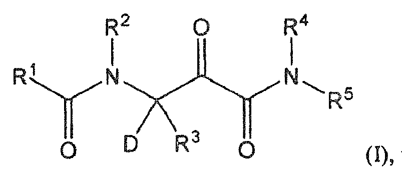 wherein:
D denotes a deuterium atom;
R¹ is 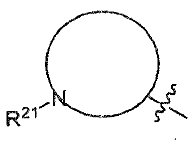 in which 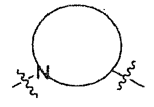 is an optionally substituted monocyclic azaheterocyclyl or optionally substituted multicyclic azaheterocyclyl, or optionally substituted multicyclic azaheterocyclenyl wherein the unsaturatation is in the ring distal to the ring bearing the R²¹ moiety and to which the -C(O)-N(R²)-CDR³-C(O)-C(O)-NR⁴R⁵ moiety is attached;
R²¹ is Q³-W³-Q²-W²-Q¹; wherein
Each of W² and W³ is independently a bond, -CO-, -CS-, -C(O)N(Q⁴) -CO₂-, -O-, -N(Q⁴)-C(O)-N(Q⁴)-, -N(Q⁴)-C(S)-N(Q⁴)-, -OC(O)NQ⁴-, -S-, -SO-, -SO₂-,--N(Q⁴)SO₂-, -N(Q⁴)SO₂N(Q⁴)-, and hydrogen when any of W² and W³ is the terminal group;
Each of Q¹, Q², and Q³ is independently a bond, an optionally substituted aliphatic, an optionally substituted heteroaliphatic, an optionally substituted cycloaliphatic, an optionally substituted aryl, an optionally substituted heteroaryl, an optionally substituted aralkyl, or an optionally substituted heteroaralkyl; or hydrogen when any of Q³, Q², or Q¹ is the terminal group, provided that Q² is not a bond when both W³ and W² are present; and
Each of R², R³, and R⁴, independently, is H or a C₁₋₆ alkyl; and
R⁵ is H, alkyl, cycloalkyl, aryl optionally substituted with 1-4 alkyl groups, alkylaryl, aryl, amino optionally substituted with 1 or 2 alkyl groups.

2. The compound of claim 1, wherein R ²¹ is 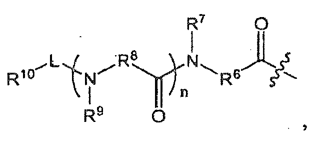 in which
each of R⁶ and R⁸ is independently
a bond; or
optionally substituted (1,1- or 1,2-)cycloalkylene; or
optionally substituted (1,1- or 1,2-)heterocyclylene; or
methylene or ethylene, substituted with one substituent selected from the group consisting of an optionally substituted aliphatic group, an optionally substituted cyclic group and an optionally substituted aromatic group, and wherein the methylene or ethylene is further optionally substituted with an aliphatic group substituent;
each of R⁷, R⁹ and R¹¹ is independently hydrogen or optionally substituted aliphatic group;
R¹⁰ is an optionally substituted aliphatic group, optionally substituted cyclic group or optionally substituted aromatic group;
L is -C(0)-, -OC(O)-, -NR¹¹(O)-, -S(O)₂-, -NR¹¹S(O)₂-, or a bond;
n is 0 or 1.

3. The compound of claim 2, wherein n is 1_{,} and/or wherein R⁶ is methylene substituted with one substituent selected from the group consisting of an optionally substituted aliphatic group, an optionally substituted cyclic group, and an optionally substituted aromatic group with R⁶ optionally being methylene substituted with isobutyls and/or wherein R⁷ is hydrogen, and/or wherein R⁸ is methylene substituted with one substituent selected from the group consisting of an optionally substituted aliphatic group, an optionally substituted cyclic group, and an optionally substituted aromatic group, with R⁸ optionally being methylene substituted with an optionally substituted cyclic group and/or methylene substituted with cyclohexyl, and/or wherein R⁹ is hydrogen, and/or wherein L is -CO; and/or wherein R¹⁰ is an optionally substituted aromatic group, said optionally substituted aromatic group being optionally selected from the group.
consisting of 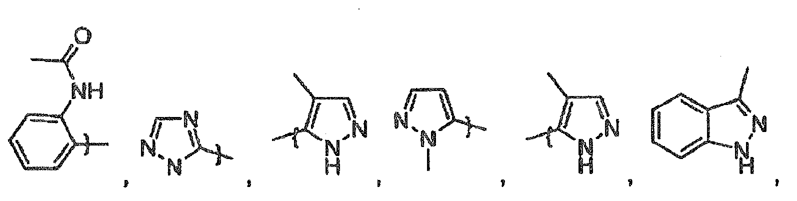 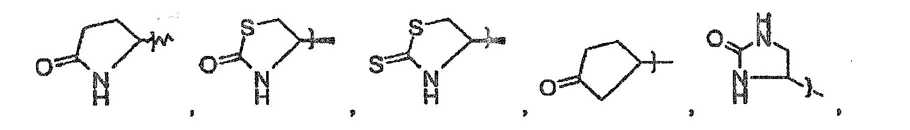 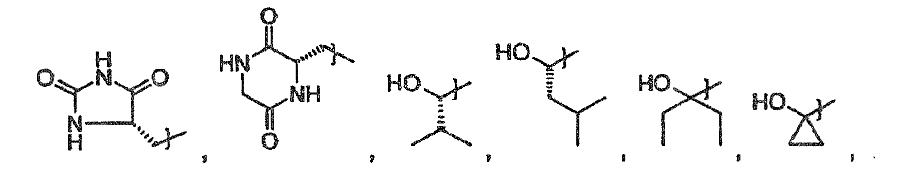 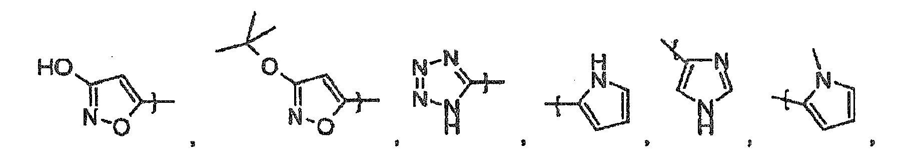 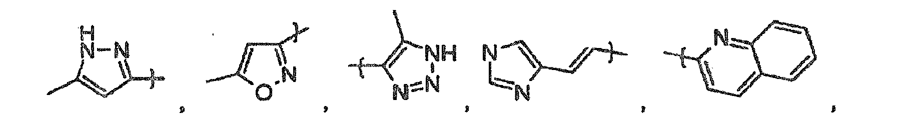 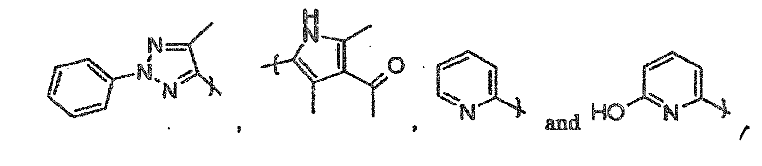 or wherein R¹⁰ is optionally substituted pyrazinyl, or wherein R¹⁰ is 2-pyrazinyl.

4. The compound of claim 2, wherein 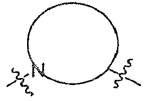 is substituted monocyclic azaheterocyclyl, or wherein 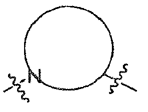 is pyrrolidinyl substituted at C-3 position with heteroaryloxy, wherein the heteroaryl, is further optionally substituted with 1-4 halo groups, or wherein 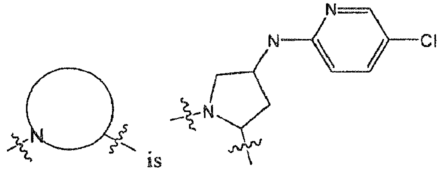 or 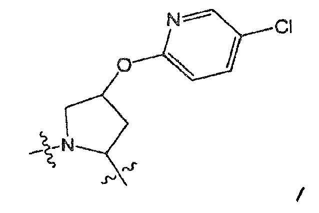 or or wherein 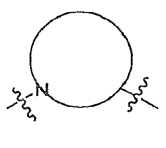 is optionally substituted multicyclic azaheterocyclyl, or wherein 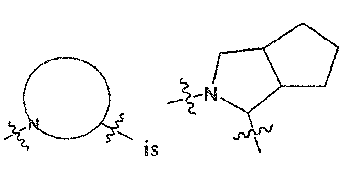 or 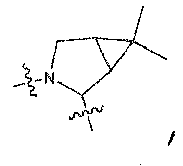 or or wherein 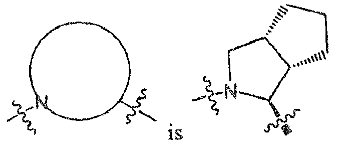 or

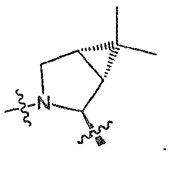

5. The compound of claim 2, wherein R² is hydrogen, each of R⁴ and R⁵ independently is hydrogen or cyclopropyl, and/orwherein R3 is propyl , and/or wherein n is 0, and/or wherein n is 0, and/or wherein L is -NR¹¹C(O)- and R¹¹ is hydrogen, and/or wherein R¹⁰ is an optionally substituted aliphatic group, said optionally substituted aliphatic group optionally being t-butyl

6. The compound of claim 2, wherein the compound is

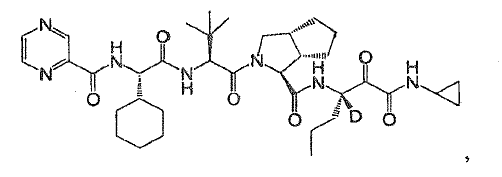

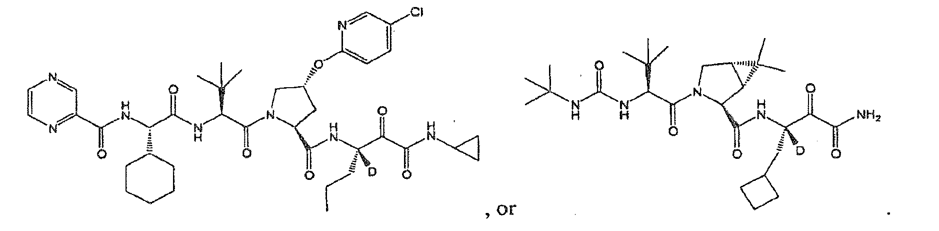

7. The compound of claim 2, wherein 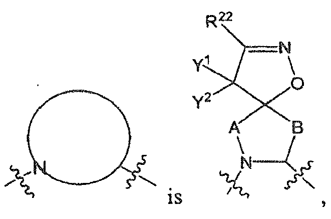 in which
A is -(CHX¹)ₐ-;
B is -(CHX² )_{b-;}
a is 0 to 3;
b is 0 to 3, provided that a + b is 2 or 3;
each of X¹ and X² is independently selected from hydrogen, optionally substituted C₁₋₄ aliphatic, and optionally substituted aryl;
each of Y¹ and Y² is independently hydrogen, optionally substituted aliphatic, optionally substituted aryl, amino, or -OQ⁴; wherein each Q⁴ is independently hydrogen or an optionally substituted aliphatic;
R²² is an optionally substituted aliphatic, an optionally substituted heteroaliphatic, an optionally substituted cycloaliphatic, an optionally substituted heterocycloaliphatic, an optionally substituted aryl, or an optionally substituted heteroaryl; and, optionally wherein R²¹ is optionally substituted alkyl carbonyl, R²¹ optionally being aminoalkylcarbonyl, haloalkylcarbonyl, arylalkylcarbonyl, arylalkylcarbonyl, cycloaliphaticalkylcarbonyl, or heterocycloaliphaticalkylcarbonyl, each of which is optionally substituted with 1-3 substituents, or R²¹ optionally being heterocyloalkyl-oxycarbonylamino-alkylcarbonyl, beteroaryl-carbonylamino-alkyl-carbonylamino-alkyl-carbonyl, bicycloaryl-sulfonylamino-alkylearbonyl, aryl-alkoxy-earbonylamino-alkyl-ewbonyl, alkyl-carbohylamino-alkyl-carbonyl, aligahatic-oxycarbonylarnino-alkyl-carbonyl, cycloaliphatic-alkyl-aminocarbonylamino-alkyl-carbonyl, cycloalighatic-alkyl-earbonylannino-alkyl-carbonyl, heteroaryl-carbonylamino-alkyl-carbonylamino-alkyl-carbonyl, alkyl-aminocarbonylamino-alkyl-carbonyl, or bicycloaryi-aminocarbonylamino-alkyl-carbonyl, each of which is optionally substituted with 1-3 substituents; or, optionally, wherein R²¹ is 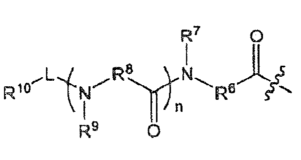 in which
each of R⁶ and R⁸ is independently
a bond; or
optionally substituted (1,1- or 1,2-)cycloalkylene; or
optionally substituted (1,1- or 1,2-)heterocyclylene; or
methylene or ethylene, substituted with one substituent selected from the group consisting of an optionally substituted aliphatic group, an optionally substituted cyclic group and an optionally substituted aromatic group, and wherein the methylene or ethylene is further optionally substituted with an aliphatic group substituent;
each of R⁷, R⁹, and R¹¹ is independently hydrogen or optionally substituted aliphatic group;
R¹⁰ is an optionally substituted aliphatic group, optionally substituted cyclic group or optionally substituted aromatic group;
L is -C(O)-, -OC(O)-, -NR¹¹C(O)-, -S(O)₂-, -NR¹¹S(O)₂-, or a bond;
n is 0 or 1; and/or, optionally, wherein R²² is an optionally substituted aliphatic, optionally substituted heteroaliphatic, optionally substituted cycloaliphatic, optionally substituted heterocycloaliphatic, optionally substituted aryl, or optionally substituted heteroaryl R²² optionally being optionally substituted phenyl, optionally substituted naphthyl, optionally substituted anthracenyl, optionally substituted naphthalene, or optionally substituted anthracene; and/or, optionally, wherein each of X¹, X², Y¹, and Y² is hydrogen, each of a and b is 1, with R²² optionally being an optionally substituted aliphatic, optionally substituted heteroaliphatic, optionally substituted cycloaliphatic, optionally substituted heterocycloaliphatic, optionally substituted aryl, or optionally substituted heteroaryl, or with R²² optionally being an optionally substituted phenyl, optionally substituted phenyl, optionally substituted naphthyl, optionally substituted anthracenyl, optionally substituted naphthalene, or optionally substituted anthracene, and, optionally, wherein R²¹ is heterocycloalkyl-oxycarbonylamino-alkylcarbonyl, beteroar-yl-carbonylamino-alkyl-carbonylamino-alkyl-carbonyl, bicycloaryl-sulfonylamino-alkylcarbonyl, aryl-alkoxy-carbonylamino-alkyl-carbonyl, alkyl-carbonylamino-alkyl-carbonyl, aliphatic-oxycarbonylamino-alkyl-carbonyl, cycloaliphatic-alkyl-aminocarbonylamino-alkyl-carbonyl, cycloaliphatic-alkyl-carbonylamino-alkyl-carbonyl, heteroaryl-carbonylamino-alkyl-carbonylamino-alkyl-carbonyl, alkyl-aminocarbonylamino-alkyl-carbonyl, or bicycloaryl-azninocarbonylamino-alkyl-carbonyl, each of which is optionally substituted with 1-3 substituents; and/or wherein

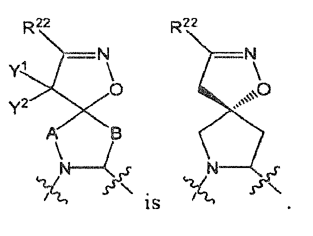

8. The compound of claim 7, wherein the compound is of the structure:

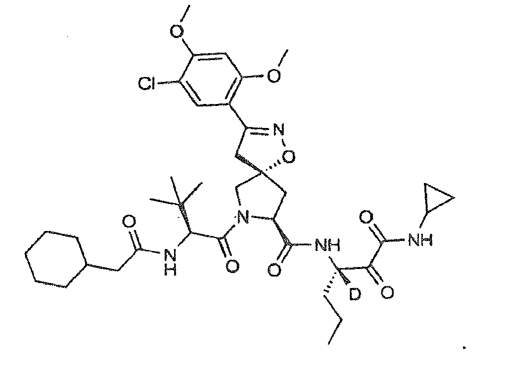

9. The compound of claim 1, wherein the deuterium enrichment is at least 50% in the compound_{.} or wherein the deuterium enrichment is at least 80%, or wherein the deuterium enrichment is at least 90%, or wherein the deuterium enrichment is at least 99%.

10. A pharmaceutical composition comprising a pharmaceutically acceptable carrier and a compound of claim 1

11. Use of a decterated isomer of a pharmaceutical agent to increase the concentration of the active isomer of a pharmaceutical agent *in vivo,* wherein, optionally, the deuterated isomer of the pharmaceutical agent is a compound of claim 1

12. A method for enhancing the bioavailability of a compound, comprising replacing a hydrogen atom that is bonded to a steric carbon atom in the compound with a deuterium atom, wherein, optionally the deuterated compound thus obtained is a compound of claim

13. A method for inhibiting HCV protease, comprising contacting HCV protease with a compound of claim 1,

14. Use of a compound of claim for treating a patient suffering from HCV infection or a condition mediated by HCV protease,

15. A process for preparing an optically enriched compound of Formula 1 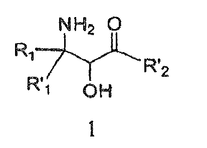 wherein:
the carbon atoms alpha and beta to the carboxy group are stereocenters;
Rᵢ is independently H, optionally substituted aliphatic, optionally substituted cycloaliphatic, optionally substituted arylaliphatic, optionally substituted heteroaliphatic or optionally substituted heteroarylaliphatic;
R'₁ is deuterium such that the deuterium enrichment is at least 50%;
R'₂ is -NHR₂ or -OE;
R₂ is H, optionally substituted aliphatic, optionally substituted cycloaliphatic, optionally substituted arylaliphatic, optionally substituted heteroaliphatic or optionally substituted heteroarylaliphatic; and
E is C₁₋₆ alkyl or benzyl;
comprising the steps of:
a) forming a salt of a compound of Formula 1, and
b) crystallizing said salt to give a compound of greater than 55% enantiomeric excess; a process for preparing a compound of Formula 1a 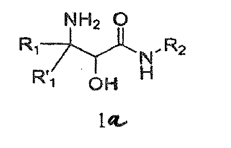
wherein:
R₁ is H, optionally substituted aliphatic, optionally substituted cycloaliphatic, optionally substituted arylaliphatic, optionally substituted heteroaliphatic or optionally substituted heteroarylaliphatic;
R'₁ is deuterium,
R₂ is H, optionally substituted aliphatic, optionally substituted cycloaliphatic, optionally substituted arylaliphatic, optionally substituted heteroaliphatic or optionally substituted heteroarylaliphatic; and
the compound of Formula 1 has an enantiomeric excess of greater than 55%, comprising the steps of:
a) oxidation of an unsaturated compound of Formula ia. 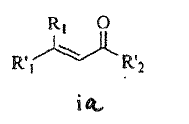 to provide a compound of formula iia 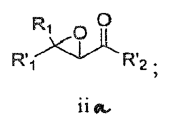
b) reacting a compound of Formula iia with an aminating reagent to provide a compound of Formula iiia. 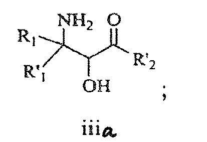
c) forming a salt of a compound of Formula iiia with an optically active organic acid;
d) crystallizing said salt to give a compound of greater than 55% enantiomeric excess; and, optionally, wherein the compound of Formula a is (2S,3S)-3-amino-3-deutero-N-cyclopropyl-2-hydroxyhexanamide, and, optionally, wherein the organic acid is L-tartaric acid or deoxycholic acid.

16. The process of preparing an optically enriched compound of formula 1 of claim 15, wherein R₁ is C₁₋₆ alkyl, and R'₂ is -NHR₂ wherein R₂ is a C₁₋₆ alkyl or C₁₋₆ cycloalkyl, alkyl or C₁₋₆ cycloalkyl, wherein R₁ optionally is propyl and R₂ optionally is cyclopropyl; and/or wherein the and/or wherein the process further comprises aminating a compound of Formula ii 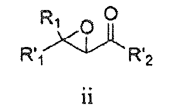 with an aminating reagent to provide a compound of Formula iii 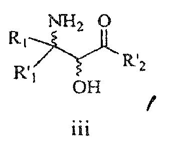 wherein the aminating reagent optionally is an azide salt and the intermediate azido compound is reduced by hydrogenation; and/or wherein the process optionally further comprises oxidizing an unsaturated compound of Formula i 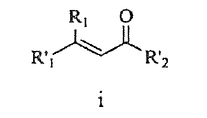 wherein R'₂ is -NHR₂ or -OE, wherein E is C₁₋₅ alkyl or optionally substituted benzyl, with an oxidizing reagent to provide a compound of Formula ii. 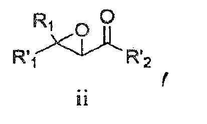 wherein the oxidizing reagent optionally comprises t-butyl hydroperoxide_{,} and, wherein the oxidizing reagent optionally further optionally chiral reagent, and wherein the oxidizing reagent optionally is mixture of samarium (III) isopropoxide, triphenyl arsine oxide, S-(-)1,1'-bi-2-naphthol and 4 Å molecular sieves, and wherein the oxidizing reagent optionally comprises urea-hydrogen peroxide in the presence of trifluoroacetic anhydride, and, optionally, wherein R'₂ is-OE or -NHR₂; and/or wherein the process optionally further comprises hydrolyzing the compound of Formula ii to give an acid and then converting the acid to an amide compound of Formula ii wherein R'₂ is -NHR₂; and/or wherein the process optionally further comprises, oxidizing a compound of Formula iv 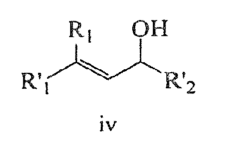 to give the compound of Formula ii,
wherein the optionally conducted by using man ganese dioxide, and/or
wherein the process optionally further comprises reducing a compound of Formula v 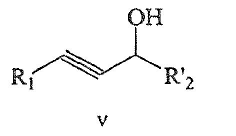 to give the compound of Formula iv,
wherein the compound optionally is reduced with Red-Al^{®} and then quenched with deuterium oxide.
